(19) Europäisches Patentamt
European Patent Office
Office européen des brevets

(11) Publication number: **0 162 576 B1**

(12) **EUROPEAN PATENT SPECIFICATION**

(45) Date of publication of patent specification: **26.07.89**

(21) Application number: **85302708.4**

(22) Date of filing: **17.04.85**

(51) Int. Cl.[4]: **C 07 D 215/26,** C 07 C 93/08, A 61 K 31/135, A 61 K 31/47 // C07C97/00, C07C95/08

(54) **Ethanolamine compounds.**

(30) Priority: **17.04.84 GB 8409908**
**11.06.84 GB 8414858**
**20.06.84 GB 8415761**
**20.06.84 GB 8415762**

(43) Date of publication of application:
**27.11.85 Bulletin 85/48**

(45) Publication of the grant of the patent:
**26.07.89 Bulletin 89/30**

(84) Designated Contracting States:
**AT BE CH DE FR GB IT LI LU NL SE**

(56) References cited:
**EP-A-0 021 636**

**CHEMICAL ABSTRACTS, vol. 88, no. 1, 2nd January 1978, page 574, no. 6752k, Columbus, Ohio, US; & JP - A - 77 83 379 (OTSUKA PHARMACEUTICAL CO., LTD.) 12-07-1977**

(73) Proprietor: **GLAXO GROUP LIMITED**
**Clarges House 6-12 Clarges Street**
**London W1Y 8DH (GB)**

(72) Inventor: **Skidmore, Ian Frederick**
**2 Wendover Drive**
**Welwyn, Hertfordshire (GB)**
Inventor: **Naylor, Alan**
**35 Layston Park**
**Royston, Hertfordshire (GB)**
Inventor: **Lunts, Lawrence Henry Charles**
**10 Wormley West End**
**Broxbourne, Hertfordshire (GB)**
Inventor: **Finch, Harry**
**19 Hensley Close**
**Hitchin, Hertfordshire (GB)**

(74) Representative: **Marchant, James Ian et al**
**Elkington and Fife Beacon House 113 Kingsway**
**London WC2B 6PP (GB)**

**EP 0 162 576 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to ethanolamine compounds having a stimulant action at $\beta_2$-adrenoreceptors, to processes for their preparation, to pharmaceutical compositions containing them and to their use in medicine.

Thus, the present invention provides compounds of the general formula (I)

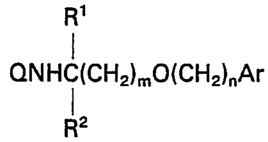

(I)

wherein

Ar represents a phenyl group optionally substituted by one or two substituents selected from halogen atoms, or $C_{1-3}$alkyl or $C_{1-3}$alkoxy groups, or by an alkylenedioxy group of formula $-O(CH_2)_pO-$ where p is 1 or 2;

$R^1$ and $R^2$ each represents a hydrogen atom or a $C_{1-3}$alkyl group with the proviso that the sum total of carbon atoms in $R^1$ and $R^2$ is not more than 4;

m is an integer from 2 to 8 and

n is an integer from 1 to 7 with the proviso that the sum total of m + n is 4 to 12;

Q represents a group of formula

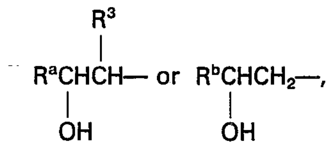

where $R^3$ represents a hydrogen atom or a $C_{1-3}$alkyl group, $R^a$ represents:

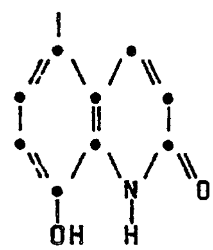

or

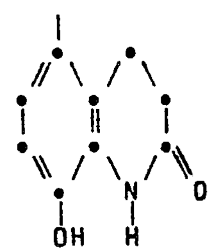

,

and $R^b$ represents:

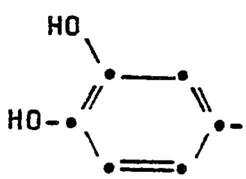

,

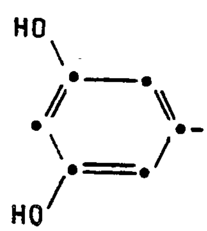

or

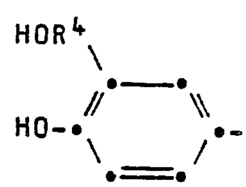

where $R^4$ represents a straight or branched $C_{2-3}$alkylene chain;
and physiologically acceptable salts and solvates (e.g. hydrates) thereof.

It will be appreciated that the compounds of general formula (I) possess one or more asymmetric carbon atoms, for example the carbon atom of the

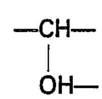

group, and, when $R^1$ and $R^2$ are different groups or $R^3$ is an alkyl group, the carbon atoms to which these are attached, and when $R^4$ is a branched alkylene chain.

The compounds according to the invention thus include all enantiomers, diasteroisomers and mixtures thereof, including racemates. Compounds in which the carbon atom in the

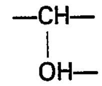

group is in the R configuration are preferred.

In the general formula (I), the chain $—(CH_2)_m—$ may be for example $—(CH_2)_3—$, $—CH_2)_4—$, $—(CH_2)_5—$, $—(CH_2)_6—$ or $—(CH_2)_7—$, and the chain $—(CH_2)_n—$ may be for example $—(CH_2)_2—$, $—(CH_2)_3—$, $—(CH_2)_4—$, $—(CH_2)_5—$ or $—(CH_2)_6—$. Preferably the total number of carbon atoms in the chains $—(CH_2)_m—$ and $—(CH_2)_n—$ is 6 to 12 inclusive and may be, for example, 7, 8, 9 or 10. Compounds wherein the sum total of m and n is 7, 8 or 9 are particularly preferred.

$R^1$ and $R^2$, for example, may each be methyl, ethyl, propyl or isopropyl groups except that if one of $R^1$ and $R^2$ is a propyl or isopropyl group, the other is a hydrogen atom or a methyl group. Thus for example $R^1$ may be a hydrogen atom or a methyl, ethyl or propyl group. $R^2$, for example, may be a hydrogen atom or a methyl group. $R^1$ and $R^2$ are each preferably a hydrogen atom or a methyl group.

A preferred group of compounds is that wherein $R^1$ and $R^2$ are both hydrogen atoms, or $R^1$ is a hydrogen atom and $R^2$ is a $C_{1-3}$alkyl group, particularly a methyl group, or $R^1$ is a methyl group and $R^2$ is a methyl group.

Examples of the substituents whcih may be present on the phenyl group represented by Ar include chlorine, bromine, iodine, or in particular fluorine or chlorine atoms or methyl, ethyl, methoxy, or ethoxy groups.

Ar is preferably an unsubstituted phenyl group.

In the definition of Q in compounds of formula (I) the group $R^3$ may be for example a hydrogen atom or a methyl, ethyl, propyl or isopropyl group, particularly a hydrogen atom or a methyl or ethyl group. The chain $R^4$ may be for example $—(CH_2)_2—$, $—(CH_2)_3—$ or

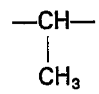

In one aspect, the invention provides a compound of formula (Ia):

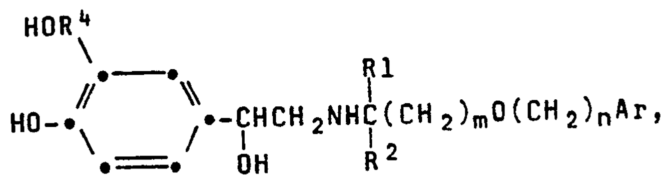

(Ia)

[wherein $R^1$, $R^2$, $R^4$, m, n and Ar are as defined for formula (I)].

In another aspect, the invention provides a compound of formula (Ib)

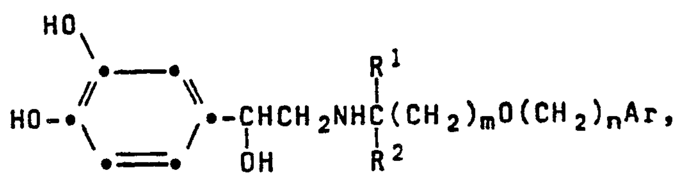

(Ib)

[wherein $R^1$, $R^2$, m, n and Ar are as defined for formula (I)].

In a further aspect, the invention provides a compound of formula (Ic)

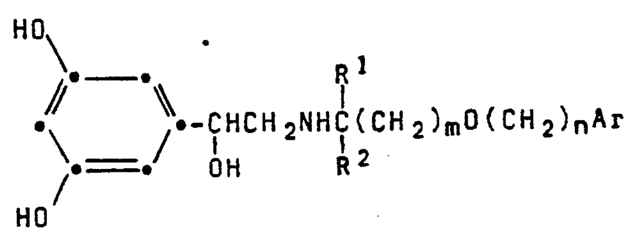

(Ic)

[wherein $R^1$, $R^2$, m, n and Ar are as defined for formula (I)].

In yet another aspect, the invention provides a compound of formula (Id):

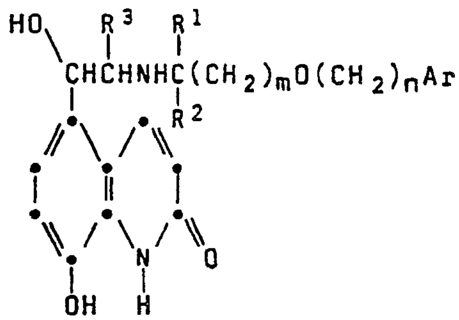

(Id)

[wherein $R^1$, $R^2$, $R^3$, $R^4$, m, n and Ar are as defined for formula (I)].

Particularly important compounds of the invention include:—

2-Hydroxy-5-[1-hydroxy-2-[[6-[2-(4-methoxyphenyl)ethoxy]hexyl]amino]ethyl]benzeneethanol;
2-Hydroxy-5-[1-hydroxy-2-[[1-methyl-6-(2-phenylethoxy)hexyl]amino]ethyl]benzeneethanol;
5-[1-Hydroxy-2-[[1-methyl-6-(2-phenylethoxy)hexyl]amino]ethyl]-1,3-benzenediol;
5-[1-Hydroxy-2-[[6-(4-phenylbutoxy)hexyl]amino]ethyl]-1,3-benzenediol;
and the physiologically acceptable salts and solvates thereof.

Suitable physiologically acceptable salts of the compounds of general formula (I) include acid addition salts derived from inorganic and organic acids, such as hydrochlorides, hydrobromides, sulphates, phosphates, maleates, tartrates, citrates, benzoates, 4-methoxy-benzoates, 2- or 4-hydroxybenzoates, 4-chlorobenzoates, p-toluenesulphonates, methanesulphonates, ascorbates, salicylates, acetates, fumarates, succinates, lactates, glutarates, gluconates, tricarballylates, hydroxynaphthalenecarboxylates e.g. 1-hydroxy- or 3-hydroxy-2-naphthalenecarboxylates, or oleates. The compounds may also form salts with suitable bases. Examples of such salts are alkali metal (e.g. sodium and potassium), and alkaline earth metal (e.g. calcium or magnesium) salts.

The compounds according to the invention have a selective stimulant action at $\beta_2$-adrenoreceptors, which furthermore is of a particularly advantageous profile. The stimulant action was demonstrated in the isolated trachea of the guinea-pig, where compounds were shown to cause relaxation of PGF2α-induced contractions. Compounds according to the invention have shown a particularly long duration of action in this test.

The selective action of compounds of the invention was demonstrated in the rat or guinea pig, where compounds were shown to have little or no effect on isolated rat or guinea pig atria ($\beta_1$-adrenoreceptor tissues) at concentrations where they cause relaxation of $PGF_{2\alpha}$-contracted isolated trachea.

The compounds according to the invention may be used in the treatment of diseases associated with reversible airways obstruction such as asthma and chronic bronchitis.

The compounds according to the invention may also be used for the treatment of premature labour, depression and congestive heart failure, and are also indicated as useful or the treatment of inflammatory and allergic skin diseases, glaucoma, and in the treatment of conditions in which there is an advantage in lowering gastric acidity, particularly in gastric and peptic ulceration.

The invention accordingly further provides compounds of formula (I) and their physiologically acceptable salts and solvates useful in the therapy or prophylaxis of diseases associated with reversible airways obstruction in human or animal subjects.

The compounds according to the invention may be formulated for administration in any convenient way. The invention therefore includes within its scope pharmaceutical compositions comprising at least one compound of formula (I) or a physiologically acceptable salt or solvate thereof formulated for use in human or veterinary medicine. Such compounds may be presented for use with physiologically acceptable carriers or excipients, optionally with supplementary medicinal agents.

The compounds may be formulated in a form suitable for administration by inhalation or insufflation, or for oral, buccal, parenteral, topical (including nasal) or rectal administration. Administration by inhalation or insufflation is preferred.

For administration by inhalation the compounds according to the invention are conveniently delivered in the form of an aerosol spray presentation from pressurised packs, with the use of a suitable propellant, such as dichlorodifluoromethane, trichloromethane, dichlorotetrafluoroethane, carbon dioxide or other suitable gas, or from a nebuliser. In the case of a pressurised aerosol the dosage unit may be determined by providing a valve to deliver a metered amount.

Alternatively, for administration by inhalation or insufflation, the compounds according to the invention may take the form of a dry powder composition, for example a powder mix of the compound and a suitable powder base such as lactose or starch. The powder composition may be presented in unit dosage form in for example capsules or cartridges of e.g. gelatin, or blister packs from which the powder may be administered with the aid of an inhaler or insufflator.

For buccal administration the composition may take the form of tablets, drops or lozenges formulated in conventional manner.

The compounds of the invention may be formulated for parenteral administration. Formulations for injections may be presented in unit dosage form in ampoules, or in multi-dose containers with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for reconstitution with a suitable vehicle, e.g. sterile pyrogen-free water, before use.

For topical administration the pharmaceutical composition may take the form of ointments, lotions or creams formulated in a conventional manner, with for example an aqueous or oily base, generally with the addition of suitable thickening agents and/or solvents. For nasel application, the composition may take the form of a spray, formulated for example as an aqueous solution or suspension or as an aerosol with the use of a suitable propellant.

The compounds of the invention may also be formulated in rectal compositions such as suppositories or retention enemas, e.g. containing conventional suppository bases such as cocoa butter or other glyceride.

Where pharmaceutical compositions are described above for oral, buccal, rectal or topical administration, these may be presented in a conventional manner associated with controlled release forms.

A proposed daily dosage of active compound for the treatment of man is 0.0005 mg to 100 mg, which may be conveniently administered in one or two doses. The precise dose employed will of course depend on the age and condition of the patient and on the route of administration. Thus a suitable dose for administration by inhalation is 0.0005 mg to 10 mg, for oral administration is 0.02 mg to 100 mg, and for parenteral administration is 0.001 mg to 2 mg.

The compounds according to the invention may be prepared by a number of processes, as described in the following wherein Ar, Q, $R^a$, $R^b$, $R^1$, $R^2$, $R^3$, $R^4$, m and n are as defined for general formula (I) unless otherwise specified. In the general processes described below the final step in the reaction may be the removal of a protecting group.

According to one general process (1), a compound of general formula (I) may be obtained by reaction of an amine of general formula (II)

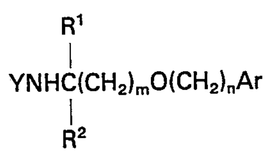

(II)

(wherein Y is a hydrogen atom or a group convertible thereto by catalytic hydrogenation) with a compound of formula

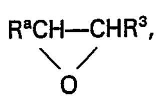, 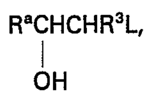, 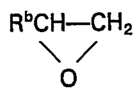 or 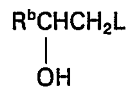

(wherein L represents a leaving group, for example a halogen atom such as chlorine, bromine or iodine, or a hydrocarbylsulphonyloxy group such as methanesulphonyloxy or p-toluenesulphonyloxy followed by removal of any protecting groups where present, as described hereinafter.

The reaction may be effected in the presence of a suitable solvent for example an alcohol, such as ethanol, a halogenated hydrocarbon e.g. chloroform, a substituted amide e.g. dimethylformamide or an ether such as tetrahydrofuran or dioxan at a temperature from ambient to the reflux, optionally in the presence of a base such as an organic amine e.g. diisopropylethylamine or an inorganic base such as sodium carbonate.

Suitable Y groups convertible into a hydrogen atom include arylmethyl groups such as benzyl, benzyhydryl, or α-methylbenzyl. Such groups may be removed by hydrogenolysis in the presence of a metal catalyst (e.g. palladium on charcoal).

In another general process (2), a compound of general formula (I) may be prepared by alkylation. Conventional alkylation procedures may be used.

Thus, for example, in one process (a), a compound of general formula (I) in which $R^1$ is a hydrogen atom may be prepared by alkylation of an amine of general formula (III)

$$QNR^5R^6 \quad (III)$$

(wherein $R^5$ is a hydrogen atom or a protecting group and $R^6$ is a hydrogen atom) followed by removal of any protecting group where present.

The alkylation (a) may be effected using an alkylating agent of general formula (IV):

$$LCH(CH_2)_mO(CH_2)_nAr \qquad (IV)$$
|
$R^2$

(wherein L is as previously defined).

The alkylation is preferably effected in the presence of a suitable acid scavenger, for example, inorganic bases such as sodium or potassium carbonate, organic bases such as triethylamine, diisopropylethylamine or pyridine, or alkylene oxides such as ethylene oxide or propylene oxide. The reaction is conveniently effected in a solvent such as acetonitrile or an ether e.g. tetrahydrofuran or dioxan, a ketone e.g. butanone or methyl isobutyl ketone, a substituted amide e.g. dimethylformamide or a chlorinated hydrocarbon e.g. chloroform at a temperature between ambient and the reflux temperature of the solvent.

According to another example (b) of an alkylation process, a compound of general formula (I) in which $R^1$ represents a hydrogen atom may be prepared by alkylation of an amine of general formula (III), as previously defined except that $R^6$ is a hydrogen atom or a group convertible thereto under the reaction conditions, with a compound of general formula (V):

$$R^2CO(CH_2)_mO(CH_2)_nAr \qquad (V)$$

in the presence of a reducing agent, followed when necessary by removal of any protecting groups.

Suitable reducing agents include hydrogen in the presence of a metal catalyst such as platinum, platinum oxide, palladium, Raney nickel or rhodium, on a support such as charcoal, using an alcohol, e.g. ethanol or an ester e.g. ethyl acetate or an ether e.g. tetrahydrofuran, or water, as reaction solvent, or a mixture of solvents, e.g. a mixture of two or more of those just described at normal or elevated temperature and pressure, for example from 20 to 100°C and from 1 to 10 atmospheres. Alternatively when one or both of $R^5$ and $R^6$ are hydrogen atoms, the reducing agent may be a hydride such as diborane or a metal hydride such as sodium borohydride, sodium cyanoborohydride or lithium aluminium hydride. Suitable solvents for the reaction with these reducing agents will depend on the particular hydride used, but will include alcohols such as methanol or ethanol, or ethers such as diethyl ether or *tert*-butyl methyl ether, or tetrahydrofuran.

Where it is desired to use a protected intermediate of general formula (III) it is particularly convenient to use hydrogen and a metal catalyst as described above with protecting groups $R^5$ and $R^6$ which are capable of being converted to a hydrogen atom under these reducing conditions. Suitable protecting groups of this type include arylmethyl groups such as benzyl, benzhydryl and α-methylbenzyl.

In a third general process (3), a compound of general formula (I) may be prepared by reduction. Thus, in one example a compound of general formula (I) may be prepared by reducing an intermediate ketone of formulae (VIa) or (VIb):

$R^3$ $R^1$
| |
$$R^aCOCHNR^5C(CH_2)_mO(CH_2)_nAr \qquad (VIa)$$
|
$R^2$

$R^1$
|
$$R^bCOCH_2NR^5C(CH_2)_mO(CH_2)_nAr \qquad (VIb)$$
|
$R^2$

[wherein $R^5$ is as defined for general formula (III)] followed where necessary by removal of any protecting groups.

The reduction may be effected using reducing agents conventionally employed for the reduction of ketones, for example hydrogen in the presence of a metal catalyst. Alternatively the reducing agent may be for example a hydride such as diborane or a metal hydride such as lithium aluminium hydride, sodium bis(2-methoxyethoxy)aluminium hydride, sodium borohydride or aluminium hydride. The reaction may be effected in a solvent, where appropriate an alcohol e.g. methanol or ethanol, or an ether such as tetrahydrofuran, or a halogenated hydrocarbon such as dichloromethane.

In another process, a compound of general formula (I) wherein $R^4$ is —$CH(CH_3)$— may be prepared by reaction of an aldehyde of general formula (VII):

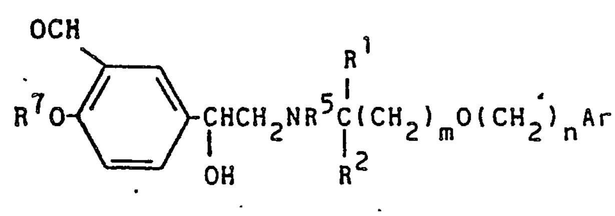

(VII)

(wherein $R^5$ is as previously defined and $R^7$ is a hydrogen atom or a protecting group) with a Grignard reagent such as methyl magnesium chloride in a solvent such as tetrahydrofuran, followed when necessary by removal of any protecting groups. Suitable protecting groups $R^7$ are tetrahydropyranyl or aralkyl groups such as benzyl, α-methylbenzyl, diphenylmethyl or triphenylmethyl. When $R^7$ is a tetrahydropyranyl group this may be cleaved by hydrolysis under acidic conditions. When $R^7$ is an aralkyl group this may be cleaved by hydrogenolysis as previously described for the groups $R^5$ and $R^6$.

It is also possible to prepare a compound of general formula (I) by a process comprising interconversion of another compound of general formula (I).

In one example, a compound of formula (I) in which $R^a$ is the group:

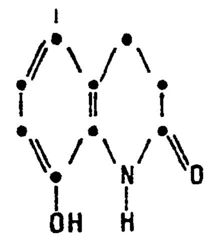

may be prepared by reduction of a corresponding compound in which $R^a$ is the group:

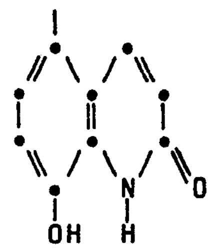

using for example hydrogen in the presence of a metal catalyst.

In the general processes described above, the compound of formula (I) obtained may be in the form of a salt, conveniently in the form of a physiologically acceptable salt. Where desired, such salts may be converted to the corresponding free bases using conventional methods.

Physiologically acceptable salts of the compounds of general formula (I) may be prepared by reacting a compound of general formula (I) with an appropriate acid or base in the presence of a suitable solvent such as acetonitrile, acetone, chloroform, ethyl acetate or an alcohol, e.g. methanol, ethanol or iso-propanol.

Physiologically acceptable salts may also be prepared from other salts, including other physiologically acceptable salts, of the compounds of general formula (I), using conventional methods.

When a specific enantiomer of a compound of general formula (I) is required, this may be obtained by resoluton of a corresponding racemate of a compound of general formula (I) using conventional methods.

Thus, in one example an appropriate optically active acid may be used to form salts with the racemate of a compound of general formula (I). The resulting mixture of isomeric salts may be separated for example by fractional crystallisation, into the diastereoisomeric salts from which the required enantiomer of a compound of general formula (I) may be isolated by conversion into the required free base.

Alternatively, enantiomers of a compound of general formula (I) may be synthesised from the appropriate optically active intermediates using any of the general processes described herein.

Specific diasteroisomers of a compound of formula (I) may be obtained by conventional methods, for example by synthesis from an appropriate asymmetric starting material using any of the processes described herein, or by conversion of a mixture of isomers of a compound of general formula (I) into appropriate diastereoisomeric derivatives e.g. salts which then can be separated by conventional means e.g. by fractional crystallisation. Alternatively, in some instances a specific diastereoisomer may be obtained through the use of particular process conditions, for example the diastereoisomers obtained from ketones of formula (VIa) where $R^3$ is an alkyl group may be determined by the method of reduction chosen. Racemates of diastereoisomers may be obtained by conventional methods of separation e.g. fractional crystallisation of a mixture of isomers of compounds of formula (I) or the salts thereof.

The intermediate compounds of formula

$$R^aCHCHR^3L \text{ and } R^bCHCH_2L$$
$$\quad OH \qquad\qquad\qquad OH$$

may be prepared from the corresponding haloketones $R^aCOCHR^3Hal$ and $R^bCOCH_2Hal$ by reduction using

for example a metal hydride such as sodium borohydride in a solvent such as ethanol. The halogen atom may be displaced to yield intermediates where L is a leaving group other than a halogen atom.

Compounds of formula

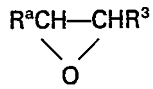 and 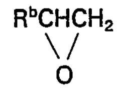

may be prepared from the corresponding compounds

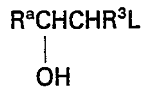 and 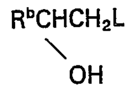

by treatment with base, for example an amine, which may be for example a compound of general formula (II), or an inorganic base such as sodium hydroxide in a solvent such as ethanol.

Intermediates of formula (III) and intermediate ketones of formulae $R^aCOCHR^3Hal$ and $R^bCOCH_2Hal$ are either known compounds or may be prepared by analogous methods to those used for the preparation of the known compounds.

Intermediate ketones of formulae (VIa) and (VIb) may be prepared from ketones of formulae $R^aCOCHR^3Hal$ and $R^bCOCH_2Hal$ by reaction with an amine of formula (II). The reaction may be effected in a cold or hot solvent, for example tetrahydrofuran, *tert*-butyl methyl ether, dioxan, chloroform, dimethylformamide, acetonitrile or a ketone such as butanone or methylisobutylketone, or an ester, for example ethyl acetate optionally in the presence of a base such as diisopropylethylamine, sodium carbonate or other acid scavenger such as propylene oxide.

Intermediate aldehydes of formula (VII) may be prepared by oxidation of an alcohol of formula (VIII):

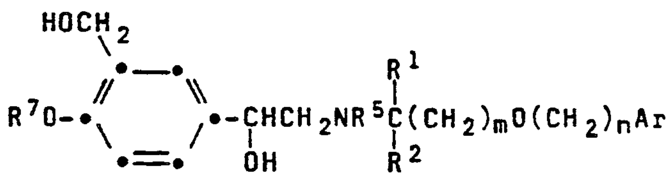 (VIII)

using an oxidising agent such as activated manganese dioxide in a solvent such as dichloromethane.

Intermediates of formulae (II), (IV), (V) and (VIII) may be prepared by the general methods described in U.K. Patent Specification No. 2140800A.

The following examples illustrate the invention. Temperatures are in °C. Thin layer chromatography (T.l.c.) was carried out over $SiO_2$. "Dried" refers to drying using magnesium sulphate except where otherwise stated.

The following abbreviations are used: EA — ethyl acetate; ER — diethyl ether, DMF — dimethylformamide; THF — tetrahydrofuran; [FCS] — flash column chromatography on silica (Merck 9385); [C] — column chromatography on silica (Merck 9385). The preparation of the following intermediates is described in U.K. Patent Specification 2140800A.

### Intermediate 1

7-[2-(Phenylethoxy)]-2-heptanone

### Intermediate 2

N-[6-(3-Phenylpropoxy)hexyl]benzenemethanamine hydrobromide

### Intermediate 7

4-Hydroxy-α[1]-[(phenylmethyl)-6-[3-(phenylpropoxy)hexyl]amino]methyl]-1,3-benzenedimethanol

### Intermediate 10

[4-[(5-Bromopentyl)oxy]butyl]benzene

### Intermediate 11

1-[2-[(6-Bromohexyl)oxy]ethyl]-4-methoxybenzene

### Intermediate 17

N-[6-(4-Phenylbutoxy)hexyl]benzenemethanamine

### Intermediate 21

[4-[(6-Bromohexyl)oxy]butyl]benzene

Intermediate 23

[2-[(6-Bromohexyl)oxy]ethyl]benzene

Intermediate 31

1,1-Dimethyl-6-(2-phenylethoxy)hexamine

Intermediate 3

1-[3-[2-(Acetyloxy)ethyl]-4-hydroxyphenyl]-2-bromoethanone

A solution of bromine (0.8 ml, 2.5 g) in chloroform (20 ml) was added over 20 min to a stirred solution of 1-[3-[2-(acetyloxy)ethyl]-4-hydroxyphenyl]ethanone (3.26 g) in chloroform (50 ml) containing a few drops of 45% hydrogen bromide in acetic acid. The red solution was diluted with ER (200 ml) washed with water (100 ml), 8% sodium bicarbonate (100 ml), brine (100 ml), dried and evaporated to give the *title compound* as an oil which solidified on standing to give a pale pink solid (4.1 g) m.p. 89—92°. Two recrystallisations from chloroform gave a sample with m.p. 98—99°C.

Intermediate 4

2-Bromo-1-[4-hydroxy-3-(2-hydroxyethyl)phenyl]ethanone

A solution of Intermediate 3 (3.5 g) in methanol (40 ml) was diluted with 2M hydrochloric acid (10 ml) and the mixture was refluxed for 1 h. The mixture was evaporated *in vacuo*, to remove most of the methanol, and the residue was diluted with water (50 ml) and extracted with ER (2 × 50 ml). The extract was washed with 8% sodium bicarbonate (50 ml), brine (50 ml), dried and evaporated to give the *title compound* as an oil which solidified on standing to give a buff solid (2.05 g) m.p. 124—125°. Recrystallisation from chloroform gave a sample with m.p. 130—131°.

Intermediate 5

1-[4-Hydroxy-3-(2-hydroxyethyl)phenyl]-2-[(phenylmethyl)[6-(3-phenylpropoxy)hexyl]amino]ethanone

A mixture of Intermediate 4 (1.0 g), Intermediate 2 (1.25 g) and N,N-diisopropylethylamine (0.8 g) in chloroform (10 ml) was stirred at 23° for 18 h. The mixture was diluted with ER (60 ml) and washed with water (2 × 25 ml), brine (20 ml), dried and evaporated to give an oil. Purification by [FCS] eluting with ER afforded the *title compound* as a viscous yellow oil (0.95 g). T.l.c. (ER) Rf 0.63.

Intermediate 6

N-[1-Methyl-6-(2-phenylethoxy)hexyl]benzenemethanamine

A solution of Intermediate 1 (11.7 g) and benzylamine (5.35 g) in toluene (50 ml) was stirred and heated under nitrogen for 2 h. The solution was hydrogenated at atmospheric pressure and room temperature over a pre-reduced 5% platinum oxide on carbon catalyst (1.5 g) in ethanol (50 ml) until the uptake of hydrogen ceased. The catalyst was removed by filtration through Hyflo® and the solvent evaporated *in vacuo* at 50°. The crude product was purified by [FCS] elution with 5% ethanol/toluene affording the *title compound* as a colourless oil (10.8 g). For analytical purposes, a portion was converted to the fumarate salt by treating the amine (325 mg) with a solution of fumaric acid (116 mg) in a mixture of ER (10 ml) and methanol (2 ml). The solution was evaporated *in vacuo* at 40° to yield a viscous oil which when triturated with ER (10 ml) afforded the fumarate saot of the *title compound* as a white powder. Recrystallisation from EA gave a colourless crystalline powder (335 mg) m.p. 91—94°.

Intermediate 8

2-Hydroxy-5-[1-hydroxy-2[(phenylmethyl)[6-(3-phenylpropoxy)hexyl]amino]ethyl]benzaldehyde

Activated manganese dioxide (*ca* 10 g) was added in 2 g portions to a stirred solution of Intermediate 7 (2.5 g) in dichloromethane (100 ml). Five minutes after each addition the reaction was monitored by t.l.c. (ER) and no more manganese dioxide was added when most of the starting material (Rf 0.63) had been converted into the product (Rf 0.39). The mixture was filtered through Hyflo® and the filtrate was evaporated to give an oil which was purified by [FCS] eluting with ER-cyclohexane (1:1) to give the *title compound* as a yellow oil (1.5 g). T.l.c. (ER-cyclohexane 1:1) Rf 0.63.

Intermediate 9

4-Hydroxy-$\alpha^3$-methyl-$\alpha^1$[[(phenylmethyl)[6-(3-phenylpropoxy)hexyl]amino]methyl]1,3-benzenedimethanol

A solution of methyl magnesium chloride in THF (2.9 M, 5 ml) was added dropwise to a stirred solution of Intermediate 8 (1.1 g) in dry THF (20 ml) at 23° undr nitrogen. The mixture was stirred at 23° for 0.5 h, diluted with saturated aqueous ammonium chloride (80 ml) and extracted with EA (2 × 50 ml). The organic phase was washed with brine (25 ml), dried and evaporated to give the *title compound* as a yellow oil (1.1 g). T.l.c. (ER) Rf 0.71.

Intermediate 12

N-[5-(4-Phenylbutoxy)pentyl]benzemethanamine

Intermediate 10 (4.0 g) was added dropwise to benzylamine (20 ml) at 110°. The solution was heated at 110—120° for 90 min, cooled, hydrochloric acid (2 M; 125 ml) was added and the mixture was extracted

with EA (2 × 100 ml). The organic extract was washed with aqueous sodium carbonate (100 ml) and brine (100 ml), dried and evaporated. The residue was distilled to give the *title compound* as a colourless oil (3.3 g), b.p. 190—195°/0.01 mmHg). T.l.c. (cyclohexane-ER 1:1) Rf 0.25.

Intermediate 13

N-[6-[2-(4-Methoxyphenyl)ethoxy]hexyl]benzenemethanamine hydrochloride

Intermediate 11 (6 g) and benzylamine (10.2 g) were stirred together at 120° for 3 h. The mixture was cooled, diluted with ER (200 ml) and washed with 2N hydrochloric acid (200 ml). Both phases were filtered to afford the crude product which was dried *in vacuo* at 40° and recrystallised from EA-hexane to give the *title compound* as a white solid (5.52 g), m.p. 109—110°.

Intermediate 14

1-[4-Hydroxy-3-(2-hydroxyethyl)phenyl]-2-[(phenylmethyl)[5-(4-phenylbutoxy)pentyl]amino]ethanone

Intermediate 4 (1 g), Intermediate 12 (1.26 g) and N,N-diisopropylethylamine (1 g) in THF (15 ml) were stirred at reflux under nitrogen for 5 h, and left at room temperature overnight. The mixture was diluted with dichloromethane (30 ml), washed with 2N hydrochloric acid (10 ml), dried ($Na_2SO_4$) and evaporated *in vacuo* to give an oil. Purification by [FCS] (triethylamine deactivated silica) eluting with toluene-ethanol (20:1) gave a yellow oil (1.49 g). T.l.c. triethylamine deactivated silica (Toluene-ethanol 100:5) Rf 0.18.

Intermediate 15

1 - [4 - Hydroxy - 3 - (2 - hydroxyethyl)phenyl] - 2 - [6 - [(4 - methoxyphenyl)ethoxy]hexyl](phenylmethyl)amino]ethanone (1.09 g). T.l.c. (toluene-ethanol 0.88 ammonia soln 39:10:1) Rf 0.45 was prepared in a similar manner to Intermediate 14 from Intermediate 4 (1 g) and Intermediate 13 (1.32 g).

Intermediate 16

1 - [4 - Hydroxy - 3 - (2 - hydroxyethyl)phenyl] - 2 - [[1 - methyl - 6 - (2 - phenylethoxy)hexyl](phenylmethyl)amino]ethanone, (0.61 g) was prepared in a similar manner to Intermediate 14 from Intermediate 4 (1.2 g) and Intermediate 6 (1.26 g).

T.l.c. (Toluene:ethanol:0.88 $NH_3$ 39:10:1) Rf 0.42.

Intermediate 18

1-(3,4-Dihydroxyphenyl)-2-[[6-(4-phenylbutoxy)hexyl)[phenylmethyl amino]ethanone

A solution of α-chloro-3,4-dihydroxyacetophenone (1.5 g), Intermediate 17 (3.6 g) and *N,N*-diisopropylethylamine (1.8 g) in THF (10 ml) was kept at 23° for 4 days. ER (70 ml) was added and the mixture was washed with 8% sodium bicarbonate (4 × 50 ml), brine (50 ml), dried and evaporated to give an oil. Purification by [FCS] using cyclohexane-ER (2:3) as eluent gave the *title compound* as a yellow oil (1.2 g).

T.l.c. (ER-cyclohexanone 3:2) Rf 0.28.

Intermediate 19

N-[5-(4-(Phenylbutoxy)pentyl]benzenemethanamine

Intermediate 10 (4.0 g) was added dropwise to benzylamine (20 ml) at 110°. The solution was heated at 110—120° for 90 min and cooled. Hydrochloric acid (2M; 125 ml) was added and the mixture was extracted with EA (2 × 100 ml). The organic extract was washed with aqueous sodium carbonate (100 ml) and brine (100 ml), dried and evaporated. The residue was distilled to give the *title compound* as a colourless oil (3.3 g) b.p. 190—195°/0.01 mmHg.

T.l.c. (Cyclohexane-ER 1:1) Rf 0.25.

Intermediate 20

1-[3,4-Dihydrophenyl]-2-[[5-(4-phenylbutoxy)pentyl](phenylmethyl)amino]ethanone

A solution of 2-chloro-3′,4′-dihydroacetophenone (1.29 g), Intermediate 19 (2.26 g) and N,N-diisopropylethylamine (2.69 g) in dichloromethane (20 ml) was stirred under nitrogen for 20 h. Potassium iodide (1.15 g) was added, the mixture stirred for a further 6 h, diluted with dichloromethane (200 ml) and washed successively with 2N hydrochloric acid (100 ml), 8% sodium bicarbonate (100 ml) then dried ($Na_2SO_4$). Concentration *in vacuo* gave the crude product which was purified by [FCS] (triethylamine deactivated silica) eluting with toluene-ethanol (19:1) to give the *title compound* as a brown oil (1.95 g).

Intermediate 22

α-[[[6-(4-Phenylbutoxy)hexyl]amino]methyl]-3,5-bis(phenylmethoxy)benzenemethanol

α-(Aminomethyl)-3,5-bis(phenylmethoxy)benzenemethanol (1.56 g), Intermediate 21 (1.16 g) and N,N-diisopropylethylamine (0.94 ml) in DMT (15 ml) were stirred at 100° under nitrogen (80 ml) for 2 h. Saturated aqueous sodium bicarbonate was added and the mixture extracted with EA (3 × 100 ml). The combined extracts were washed with water (80 ml), dried and evaporated to give a yellow oil. The crude oil in EA was purified by [FCS] eluting with EA — triethylamine (99:1) to give the *title compound* as a white solid (0.7 g), m.p. 65—67°.

Intermediate 24

N-[6-(2-Phenylethoxy)hexyl]benzenemethanamine

Intermediate 23 (4.0 g) was added dropwise to benzylamine (20 ml) at 110°. The solution was heated at 110—120° for 90 min, cooled, and treated with hydrochloric acid (2M; 125 ml). The mixture was extracted with EA (2 × 100 ml) and the extract was washed with aqueous sodium carbonate (100 ml) and brine (100 ml), dried and evaporated. Distillation of the residue gave the *title compound* as a colourless oil (3.2 g) b.p. 180—190°/0.1 mmHg. T.l.c. (Cyclohexane — ER 1:1) Rf 0.2.

Intermediate 25

α-[[[6-(2-Phenylethoxy)hexyl](phenylmethyl)amino]methyl]-3,5-bis(phenoxymethoxy)benzenemethanol

A solution of 2-[3,5-bis(phenylmethoxy)phenyl]oxirane (1.0 g) and Intermediate 24 (1.0 g) in methanol (20 ml) was refluxed for 18 h and evaporated. The residue was purified by [C] eluting with cyclohexane ER (3:1). Repeated [C] eluting with chloroform gave the *title compound* as a colourless oil (0.8 g). T.l.c. (Cyclohexane — ER 3:1) Rf 0.2.

Intermediate 26

2-[[5-(4-Phenylbutoxy)pentyl](phenylmethyl)amino]-1-[3,5-bis(phenylmethoxy)phenyl]ethanone

A solution of 2-bromo-1-[3,5-bis(phenylmethoxy)phenyl]ethanone (2.45 g) Intermediate 19 (2.0 g) and N,N-diisopropylethylamine (0.774 g) in dichloromethane (20 ml) was stirred for 20 h at room temperature. ER (150 ml) was added and the mixture was washed with water, dried ($Na_2SO_4$), and evaporated. The residue was purified by [C] eluting with cyclohexane — ER (9:1) to give the *title compound* as a yellow oil (2.4 g).

T.l.c. (Cyclohexane — ER 3:1 Rf 0.4.

Intermediate 27

1-[2-(4-Bromobutoxy)ethyl]-4-fluorobenzene

A mixture of 4-fluorobenzeneethanol (10.0 g), 1,4-dibromobutane (59.0 g), aqueous sodium hydroxide (50% w/v; 40 ml) and tetrabutylammonium bisulphate (1 g) was stirred at room temperature for 20 h, diluted with water (50 ml) and extracted with ER (2 × 100 ml). The dried extract was evaporated and the residue was purified by [C] eluting with cyclohexane followed by cyclohexane-ER (19:1) to give the *title compound* as a colourless oil (17.3 g).

T.l.c. (cyclohexane — ER 9:1) Rf 0.4.

Intermediate 28

7-[2-(4-Fluorophenyl)ethoxy]-2-heptanone

A mixture of Intermediate 27 (10.0 g), acetylacetone (5.0 g), potassium iodide (8.3 g ), potassium carbonate (5.52 g) and ethanol (75 ml) was refluxed for 22 h, filtered, and evaporated. The residue was treated with ER (200 ml), filtered and evaporated. The resulting oil was purified by [C] eluting with cyclohexane — ER (4:1) to give the *title compound* as a colourless oil (4.3 g).

T.l.c. (cyclohexane — ER 3:1) Rf 0.25.

Intermediate 29

1-[[2-(4-Bromobutoxy)]ethyl]-4-methoxybenzene

4-Methoxybenzeneethanol (20 g), 1,4-dibromobutane (99.31 g), tetrabutylammonium bisulphate (5.92 g) and 50% sodium hydroxide solution (200 ml) were stirred at room temperature for 18 h. The mixture was diluted with water (300 ml) and extracted with ER (2 × 400 ml), dried and evaporated *in vacuo* to give a colourless oil (39.4 g). Purification by [FCS] eluting with cyclohexane — ER (100:0 → 90:1) gave the *title compound* as a colourless oil (30.9 g).

T.l.c. (cyclohexane — ER 9:1) Rf 0.36.

Intermediate 30

7-[2-(4-Methoxyphenyl)ethoxy]-2-heptanone

A mixture of Intermediate 29 (12.0 g), acetylacetone (6.0 g), potassium iodide (13.3 g), potassium carbonate (6.95 g), and ethanol (75 ml) was refluxed for 18 h, filtered, and evaporated. The residue was treated with ER (200 ml), filtered, and evaporated. The resulting oil was purified by [C] eluting with cyclohexane — ER (4:1) to give the *title compound* as a colourless oil (4.7 g).

T.l.c. (cyclohexane — ER 3:1) Rf 0.25.

Intermediate 32

N-[1,1-Dimethyl-6-(2-phenylethoxy)hexyl]benzenemethanamine

A solution of Intermediate 31 (2.3 g), benzaldehyde (1.27 g) and 4-toluenesulphonic acid (0.02 g) in toluene (150 ml) was refluxed for 18 h and evaporated. The residue in ethanol (50 ml) was hydrogenated over 5% platinum on charcoal (0.4 g) for 2 h, filtered and evaporated. The resulting oil was purified by [C] eluting with cyclohexane-ER (3:1) to give the *title compound* as a colourless oil (2.1 g).

T.l.c. (cyclohexane-ER 3:1) Rf 0.2.

Intermediate 33

2-[[1,1-Dimethyl-6-(2-phenylethoxy)hexyl](phenylmethyl)amino-1-[3,5-bis(phenylmethoxy)phenyl]-ethanone

A solution of 2-bromo-1-[3,5-bis(phenylmethoxy)phenyl]ethanone (2.5 g), Intermediate 32 (2.0 g) and N,N-diisopropylethylamine (0.77 g) in THF (25 ml) was stirred at room temperature for 2 h and refluxed for 20 h. ER (100 ml) was added and the resulting suspension was filtered, the filtrate was evaporated and the residue was purified by [C] eluting with cyclohexane-ER (4:1) to give the *title compound* as a pale yellow oil (1.2 g). T.l.c. (cyclohexane-ER 3:1) Rf 0.4.

Intermediate 34

α-[[[6-[2-(4-methoxyphenyl)ethoxy]hexyl](phenylmethyl)amino]methyl]-3,4-bis(phenylmethoxy)benzenemethanol

A solution of 1-bromo-2-[3,4-bis(phenylmethoxy)phenyl]ethanone (1.0 g), Intermediate 13, free base (0.83 g) and N,N-diisopropylethylamine (0.55 g) in THF (15 ml); was stirred under nitrogen for 18 h. The solvent was evaporated *in vacuo* to give an oil which was dissolved in EA (50 ml) and washed with 2N hydrochloric acid (50 ml). The aqueous phase was re-extracted with EA (20 ml), the organic phases combined, dried ($Na_2SO_4$) and evaporated *in vacuo* to give a yellow oil which was purified by [FCS] (triethylamine deactivated silica) eluting with cyclohexane-EA (1:1). The resulting yellow oil was dissolved in absolute ethanol (25 ml) and THF (5 ml), the solution treated at 0° under nitrogen with sodium borohydride (0.115 g). The mixture was stirred under nitrogen for 44 h, diluted with 2N hydrochloric acid (5 ml) and the solvent evaporated *in vacuo* at 40°. The residue was partitioned between 2N sodium bicarbonate solution (10 ml) and EA (50 ml), the aqueous layer extracted with further EA (20 ml) and the combined organic extracts dried ($Na_2SO_4$) and evaporated *in vacuo* to give a yellow oil which was purified by [FCS] eluting with cyclohexane-EA (3:1) to give the *title compound* as a yellow oil (0.96 g).

T.l.c. (cyclohexane-EA 3:1) Rf 0.24.

Example 1

2-Hydroxy-5-[1-hydroxy-2-[[6-(3-phenylpropoxy)hexyl]amino]ethyl]benzeneethanol

A solution of Intermediate 5 (0.5 g) in absolute ethanol (25 ml) was hydrogenated at room temperature and atmospheric pressure over 10% palladium on carbon and 10% platinum on carbon catalysts (0.15 g of each). When hydrogen absorption (49 ml) ceased the mixture was filtered and the filtrate evaporated to give an oil. Purification by [FCS] (triethylamine-deactivated silica) eluting with EA-methanol (9:1) afforded the *title compound* as an oil which on trituration with ER gave a white solid (0.31 g) m.p. 64—65°.

T.l.c. triethylamine-deactivated silica (EA-methanol 9:1) Rf 0.21.

Example 2

4-Hydroxy-$\alpha^3$-methyl-$\alpha^1$[[[6-(3-phenylpropoxy)hexyl]amino]methyl]-1,3-benzenedimethanol, (0.52 g) was prepared in a similar manner to Example 1 from Intermediate 9 (1.1 g).

T.l.c. triethylamine-deactivated silica (EA-methanol 9:1) Rf 0.4.

Example 3

2-Hydroxy-5-[1-hydroxy-2-[[5-(4-phenylbutoxy)pentyl]amino]ethyl]benzeneethanol

A solution of Intermediate 14 (1.36 g) in absolute ethanol (55 ml) was hydrogenated over a mixture of 10% palladium on charcoal (140 mg) and 5% platinum on charcoal (140 mg) catalysts. The mixture was filtered through hyflo and evaporated *in vacuo* to give a colourless oil (1.12 g). Trituration with ER afforded the *title compound* as a white solid (0.6 g) m.p. 65—67°.

| | | | |
|---|---|---|---|
| Analysis Found: | C, 71.95; | H, 8.68; | N, 3.45. |
| $C_{25}H_{37}NO_4$ requires: | C, 72.25; | H, 8.97; | N, 3.37%. |

Example 4

2-Hydroxy-5-[1-hydroxy-2-[[6-[2-(4-methoxyphenyl)ethoxy]hexyl]amino]ethyl]benzeneethanol (0.42 g) was prepared in a similar manner to Example 3 from Intermediate 15 (0.79 g). m.p. 84—85°.

T.l.c. (toluene-ethanol-0.88 ammonia solution 39:10:1) Rf 0.28.

Example 5

2-Hydroxy-5-[1-hydroxy-2-[[1-methyl-6-(2-phenylethoxy)hexyl]amino]ethyl]benzeneethanol hydrochloride (4:3)

A solution of Intermediate 16 (1 g) in absolute ethanol (40 ml) was hydrogenated over a mixture of pre-reduced 10% palladium on charcoal (100 mg) and 5% platinum on charcoal (100 mg) catalysts in absolute ethanol (10 ml) until the uptake of hydrogen (78 ml) ceased. The mixture was filtered through Hyflo® and evaporated *in vacuo* to give a yellow oil (0.81 g). Purification by [FCS] (triethylamine deactivated silica) eluting with toluene-ethanol (19:1) gave a yellow oil (0.81 g) which was triturated with ethereal hydrogen chloride to give the *title compound* as an off-white solid (0.29 g) m.p. 97—100°.

T.l.c. silica (Toluene:ethanol: 0.88 $NH_3$ 39:10:1) Rf 0.33.

Example 6

8-Hydroxy-5-[1-hydroxy-2-[[1-methyl-6-(2-phenylethoxy)hexyl]amino]ethyl]-2(1*H*)-quinolinone

A slurry of 8-hydroxy-5-[[(phenylmethyl)amino]acetyl]-2-(1*H*)-quinoline hydrochloride (382 mg) and Intermediate 1 (311 mg) in ethanol (20 ml) was added to a pre-reduced mixture of 10% palladium oxide on carbon (160 mg), 5% platinum oxide on carbon (160 mg), and anhydrous sodium acetate (158 mg) in ethanol (5 ml) and hydrogenated. The catalyst and solvent were removed and the residue was treated with aqueous saturated sodium bicarbonate (20 ml) and EA (15 ml). The precipitate was filtered off, dried *in vacuo*, and purified by [FCS] eluting with EA-methanol-triethylamine (73:25:2) to give a solid which was adsorbed onto silica gel (Merck 7734, 3 g) from methanol. The silica gel plug was applied to [FCS] eluting with EA-methanol-triethylamine [94:5:1 → 79:20:1) to give, after trituration with ER the *title compound* as a light yellow solid (63 mg), m.p. 134—138°. T.l.c. (Toluene-ethanol-$NH_3$, 78:20:2) Rf 0.15.

Example 7

4-[1-Hydroxy-2-[[6-(4-phenylbutoxy)hexyl]amino]ethyl]-1,2-benzenediol

A solution of Intermediate 18 (1 g) in absolute ethanol (25 ml) was hydrogenated over 10% palladium on carbon (0.15 g) and 10% platinum on carbon (0.15 g) catalysts. The mixture was filtered and evaporated to give a solid which was slurried in ER (25 ml) and filtered to give the *title catchol* as a pale-mauve solid (0.28 g) m.p. 126—7° (dec). T.l.c. triethylamine-deactivated silica (EA-methanol 1:1) Rf 0.72.

Example 8

(1R)-4-[1-Hydroxy-2-[[1-methyl-6-(2-phenylethoxy)hexyl]amino]ethyl-1,2-benzenediol.

Intermediate 1 (1.00 g), *R*-4-(2-amino-1-hydroxyethyl)-1,2-benzenediol (722 mg), acetic acid (774 mg) and methanol (18 ml) were treated with sodium cyanoborohydride (188 mg) at room temperature. The mixture was stirred for 17 h, poured into aqueous saturated sodium bicarbonate (25 ml), extracted with EA (3 × 25 ml), and the combined, dried ($Na_2SO_4$) extracts were evaporated. The residue (1.83 g) was adsorbed onto silica gel (Merck 7734, 6 g) and subjected to [FCS] eluting with EA-methanol-triethylamine (90:9:1) to give a light brown solid (0.26 g) which was further purified by chromatography as above to give the *title compound* as a red solid (67 mg), m.p. 116—119°.

| | | | |
|---|---|---|---|
| Analysis Found: | C, 69.6; | H, 8.6; | N, 3.65. |
| $C_{23}H_{33}NO_4.0.074Et_3N.0.15H_2O$ requires: | C, 70.8; | H, 8.7; | N, 3.8%. |

Example 9

4-[1-Hydroxy-2-[[5-(4-phenylbutoxy)pentyl]amino]ethyl]-1,2-benzenediol

A solution of Intermediate 20 (1.89 g) in absolute ethanol (70 ml) was hydrogenated over a mixture of 5% platinum on charcoal (0.2 g) and 10% palladium on charcoal (0.2 g) catalysts until the uptake of hydrogen ceased. The mixture was filtered through Hyflo®, evaporated *in vacuo* and the crude product was purified by [FCS] (triethylamine deactivated silica) eluting with toluene-ethanol (9:1) to give a brown oil. Trituration with ER gave the *title compound* as a brown solid (0.21 g).

| | | | |
|---|---|---|---|
| Analysis Found: | C, 68.0; | H, 8.2; | N, 3.4. |
| $C_{23}H_{33}NO_4.H_2O$ requires: | C, 67.1; | H, 8.7; | N, 3.45%. |

Example 10

5-[1-Hydroxy-2-[[6-(4-phenylbutoxy)hexyl]amino]ethyl]-1,3-benzenediol

Intermediate 22 (0.53 g) in methanol (50 ml) was hydrogenated over 10% palladium oxide on carbon (0.26 g). The catalyst was removed by filtration through hyflo and the filtrate evaporated at reduced pressure. The resulting green gum was dissolved in EA (10 ml) and adsorbed into silica gel (2 g Merck 7734). The dried silica gel plug was applied to [FCS] eluting with triethylamine in EA to give a clear gum. Co-evaporation with ER (2 × 10 ml) gave the *title compound* as a green friable solid (0.28 g) which on standing became a glass, m.p. 37—39°.

T.l.c. (EA-methanol-triethylamine, 89:10:1) Rf 0.3.

Example 11

5-[1-Hydroxy-2-[[6-(2-phenylethoxy)hexyl]amino]ethyl]-1,3-benzenediol benzoate (salt)

A solution of Intermediate 25 (0.7 g) in ethanol (10 ml) and THF (10 ml) was hydrogenated over 10% palladium on charcoal (0.1 g). The mixture was filtered and evaporated and the residue was purified by [C] eluting with EA-methanol-triethylamine (90:10:1) to give a colourless oil (0.3 g). The oil in chloroform (10 ml) was treated with benzoic acid (0.15 g) in chloroform (5 ml) and solvent was evaporated to leave a colourless gum. The gum was triturated with ER (3 × 15 ml) and dried under vacuum to give the *title compound* as a beige friable solid (0.27 g). T.l.c. (EA-methanol-$NH_3$ 90:10:1) Rf 0.15.

| | | | |
|---|---|---|---|
| Analysis Found: | C, 69.4; | H, 7.7; | N, 2.8. |
| $C_{22}H_{31}NO_4.C_7H_6O_2.0.3H_2O$ requires: | C, 69.5; | H, 7.6; | N, 2.8%. |

Example 12

5-[1-Hydroxy-2-[[1-methyl-6-(2-phenylethoxy)hexyl]amino]ethyl]-1,3-benzenediol benzoate (salt)

A solution of 3,5-bis(phenylmethoxy)-α-[[bis(phenylmethyl)amino]methyl]benzenemethanol (1.0 g)

and Intermediate 1 (0.44 g) in ethanol (30 ml) and THF (10 ml) was hydrogenated over 10% palladium on charcoal (0.2 g) and 5% platinum on charcoal (0.2 g). The mixture was filtered and evaporated and the residue was purified by [C] eluting with EA-methanol-trioethylamine (90:10:1) to give a colourless oil. A solution of the oil and benzoic acid (0.15 g) in chloroform (10 ml) was evaporated and the residue was triturated with ER (2 × 10 ml) to give the *title compound* as an off-white solid (0.42 g) m.p. 77—78°. T.l.c. (EA-methanol-$NH_3$ 9:1:0.1) Rf 0.2.

The following compound was prepared in a similar manner to Example 11:

Example 13

5-[1-Hydroxy-2-[[5-(4-phenylbutoxy)pentyl]amino]ethyl]-1,3-benzenediol benzoate (salt), 0.32 g) from Intermediate 26 (1.0 g).

T.l.c. (EA-methanol-$NH_3$ 9:1:0.1) Rf 0.15.

| | | | |
|---|---|---|---|
| Analysis Found: | C,C, 69.6; | H, 8.1; | N, 2.8. |
| $C_{23}H_{33}NO_4.C_7H_6O_2.0.5H_2O$ requires: | C,C, 69.5; | H, 7.8; | N, 2.7%. |

The following two compounds were prepared in a similar manner to Example 12:—

Example 14

5-[2-[[6-[2-(4-Fluorophenyl)ethoxy]-1-methylhexyl]amino]-1-hydroxyethyl]-1,3-benzenediol, benzoate (salt), (0.6 g) from 3,5-bis(phenylmethoxy)-α-[[bis(phenylmethyl)amino]methyl]benzenemethanol (2.2 g) and Intermediate 28 (1.0 g) m.p. 79—83°.

T.l.c. (EA-methanol-$NH_3$ (90:10:1) Rf 0.25.

Example 15

5-[1-Hydroxy-2-[[6-[2-(4-methoxyphenyl)ethoxy]-1-methylhexyl]amino]ethyl]-1,3-benzenediol, benzoate (salt), (0.75 g) from 3,5-bis(phenylmethoxy)-α-[[bis(phenylmethyl)amino]methyl]benzenemethanol (1.9 g) and Intermediate 30 (1.0 g) m.p. 74—81°.

T.l.c. (EA-methanol-$NH_3$ 90:10:1) Rf 0.2.

Example 16

5-[2-[1,1-Dimethyl-6-(2-phenylethoxy)hexyl]amino]-1-hydroxyethyl]-1,3-benzenediol, benzoate (salt), from Intermediate 33 (1.2 g), m.p. 115—119°.

T.l.c. (EA-methanol-$NH_3$ 90:10:1) Rf 0.2, in a similar manner to Example 11.

Example 17

4-[1-Hydroxy-2-[[6-[2-(4-methoxyphenyl)ethoxy]hexyl]amino]ethyl]-1,2-benzenediol

A solution of Intermediate 34 (0.85 g) in absolute ethanol (80 ml) was hydrogenated over a mixture of pre-reduced 5% platinum on charcoal (200 mg) and 10% palladium on charcoal (200 mg) catalysts in absolute ethanol (20 ml) until the uptake of hydrogen ceased (1.5 h). The solution was filtered through Hyflo® under nitrogen, the solvent evaporated *in vacuo*, and the residue triturated with ER to give the *title compound* as a greyish-blue solid (180 mg) m.p. 124—125.5° (dec).

T.l.c. (Toluene-ethanol-0.88 ammonia solution 39:10:1). Rf 0.24.

The following are examples of suitable formulations of compounds of the invention. The term "active ingredient" is used herein to represent a compound of the invention.

*Tablets*

These may be prepared by the normal methods as wet granulation or direct compression.

A. Direct Compression

| | mg/tablet |
|---|---|
| Active ingredient | 2.0 |
| Microcrystalline Cellulose USP | 196.5 |
| Magnesium Stearate BP | 1.5 |
| Compression weight | 200.0 |

The tablets are prepared by the normal methods of direct compression.

B. Wet Granulation

The active ingredient is sieved through a suitable sieve and blended with lactose, starch and pregelatinised maize starch. Suitable volumes of purified water are added and the powders are granulated.

After drying, the granules are screened and blended with the magnesium stearate. The granules are then compressed into tablets using 7 mm diameter punches.

Tablets of other strengths may be prepared by altering the ratio of active ingredient to lactose or the compression weight and using punches to suit.

C. For buccal administration

| | mg/tablet |
|---|---|
| Active ingredient | 2.0 |
| Lactose BP | 94.8 |
| Sucrose BP | 86.7 |
| | mg/tablet |
| Hydroxypropylmethylcellulose | 15.0 |
| Magnesium Stearate BP | 1.5 |
| Compression weight | 200.0 |

The active ingredient is sieved through a suitable sieve and blended with the lactose, sucrose and hydroxypropylmethylcellulose. Suitable volumes of purified water are added and the powders are granulated. After drying, the granules are screened and blended with the magnesium stearate. The granules are then compressed into tablets using suitable punches.

The tablets may be film coated with suitable film forming materials, such as hydroxylpropyl methylcellulose, using standard techniques. Alternatively the tablets may be sugar coated.

*Capsules*

| | mg/capsule |
|---|---|
| Active ingredient | 2.0 |
| * Starch 1500 | 97.0 |
| Magnesium Stearate BP | 1.0 |
| Fill weight | 100.0 |

*A form of directly compressible starch.

The active ingredient is sieved and blended with the excipients. The mix is filled into size No. 2 hard gelatin capsules using suitable machinery. Other doses may be prepared by altering the fill weight and if necessary changing the capsule size to suit.

*Syrup*

This may be either a sucrose or sucrose free presentation.

A. Sucrose Syrup

| | mg/5 ml dose |
|---|---|
| Active ingredient | 2.0 |
| Sucrose BP | 2750.0 |
| Glycerine BP | 500.0 |
| Buffer<br>Colour<br>Preservative | as required |
| Purified Water BP | to 5.0 m |

The active ingredient, buffer, flavour, colour and preservative are dissolved in some of the water and

the glycerine is added. The remainder of the water is heated to dissolve the sucrose and is then cooled. The two solutions are combined, adjusted to volume and mixed. The syrup produced is clarified by filtration.

B. Succrose-Free

| | | mg/5 ml dose |
|---|---|---|
| Active ingredient | | 2.0 mg |
| Hydroxypropyl methylceluloe USP (viscosity type 4000) | | 22.5 mg |
| Buffer, Flavour, Colour, Preservative | | as required |
| Sweetener | | |
| Purified Water BP | to | 5.0 ml |

The hydroxypropyl methylcellulose is dispersed in hot water, cooled and then mixed with an aqueous solution containing the active ingredient and the other components of the formulation. The resultant solution is adjusted to volume and mixed. The syrup is clarified by filtration.

*Meter Dose Pressurised Aerosol*

A. Suspension Aerosol

| | mg/metered dose | Per can |
|---|---|---|
| Active ingredient (micronised) | 0.100 | 26.40 mg |
| Oleic Acid BP | 0.100 | 2.64 mg |
| Trichlorofluoromethane BP | 23.64 | 5.67 g |
| Dichlorodifluoromethane BP | 61.25 | 14.70 |

The active ingredient is micronised in a fluid energy mill to a fine particle size range. The Oleic Acid is mixed with the Trichlorofluoromethane at a temperature of 10—15°C and the micronised drug is mixed into the solution with a high shear mixer. The suspension is metered into aluminium aerosol cans and suitable metering valves, delivering 85 mg of suspension are crimped onto the cans and the Dichlorofluoromethane is pressure filled into the cans through the valves.

B. Solution Aerosol

| | mg/metered dose | Per can |
|---|---|---|
| Active ingredient | 0.055 | 13.20 mg |
| Ethanol BP | 11.100 | 2.66 g |
| Dichlorotetrafluoroethane BP | 25.160 | 6.04 g |
| Dichlorodifluoromethane BP | 37.740 | 9.06 g |

Oleic acid BP, or a suitable surfactant, e.g. Span 85 (sorbitan trioleate) may also be included.

The active ingredient is dissolved in the ethanol together with the oleic acid or surfactant if used. The alcoholic solution is metered into suitable aerosol containers followed by the dichlorotetrafluoroethane. Suitable metering valves are crimped onto the containers and dichlorodifluoromethane is pressure filled into them through the valves.

*Suppositories*

| | | |
|---|---|---|
| Active ingredient | | 2.0 mg |
| * Witepsol H15 | to | 1.0 g |

*A proprietary grade of Adeps Solidus Ph. Eur.

A suspension of the active ingredient in molten Witepsol is prepared and filled, using suitable machinery, into 1 g size suppository moulds.

*Injection for Intravenous Administration*

| | | mg/ml |
|---|---|---|
| Active ingredient | | 0.5 mg |
| Sodium Chloride BP | | as required |
| Water for Injection BP | to | 1.0 ml |

Sodium chloride may be added to adjust the tonicity of the solution and the pH may be adjusted, using acid or alkali, to that of optimum stability and/or facilitate solution of the active ingredient. Alternatively suitable buffer salts may be used.

The solution is prepared, clarified and filled into appropriate size ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen or other suitable gas.

*Inhalation Cartridges*

| | | mg/cartridge |
|---|---|---|
| Active ingredient micronised | | 0.200 |
| Lactose BP | to | 25.0 |

The active ingredient is micronised in a fluid energy mill to a fine particle size range prior to blending with normal tabletting grade lactose in a high energy mixer. The powder blend is filled into No. 3 hard gelatin capsules on a suitable encapsulating machine. The contents of the cartridges are administered using a powder inhaler such as the Glaxo Rotahaler.

The stimulant action at $\beta_2$-adrenoreceptors of compounds of the invention was determined using the following:

*Guinea-Pig Tracheal Strip Preparation*

Tracheal rings were mounted in a superfusion apparatus, and continuously superfused with oxygenated physiological (Kreb's) solution containing indomethacin ($2.4 \times 10^{-6}$M) and atropine ($4 \times 10^{-7}$M) at 37° at a rate of 2 ml/min. Changes in tension of the preparation were measured using an isomeric strain gauge. Preparations were contracted for the duration of the test by the inclusion of prostaglandin $F_2\alpha$ ([$2.9 \times 10^{-6}$M) in the superfusion fluid. Two bolus dose-effect curves to the standard, isoprenaline, ($1 \times 10^{-12}$—$1 \times 10^{-9}$ moles) were obtained at the start of each test in a cumulative fashion, allowing the relaxation obtained with each to reach its own maximum before the next increment was made. On completion of this dose-effect curve, sufficient time was allowed for the tissue to recover (15—30 min). After this time, sequential concentration-effect curves were constructed for first isoprenaline and then the test compound. These were constructed as follows: a low concentration (isoprenaline $3 \times 10^{-10}$M; test compound $1 \times 10^{-10}$M) was infused until any response obtained had reached its maximum, then the infusion was stopped and the tissue allowed to recover for a maximum of 30 min. After this period the procedure was repeated using progressively increasing concentrations of agonist, and in this way, whole concentration-effect curves obtained. Potency was determined by comparison of the concentration-effect curve thus constructed with that previously obtained for isoprenaline and expressed as equipotent concentration

$$(\text{isoprenaline} = 1) \text{ i.e. } \frac{EC_{50} \text{ test compound}}{EC_{50} \text{ isoprenaline}} \text{ was calculated.}$$

Duration of action was also measured for each response, and is the time taken from stopping the infusion to 50% recovery. Graphs were drawn for duration times against response magnitude, and from

these, duration times for 50% maximum responses were determined.

The following potencies (expressed as equipotent concentration, isoprenaline = 1) were determined using the guinea-pig tracheal test described above:—

| Compound of Example No. | Potency (Isoprenaline = 1) |
|---|---|
| 5 | 0.17 |
| 6 | 0.16 |
| 8 | 0.4 |
| 12 | 0.15 |
| 14 | 0.13 |
| 17 | 0.6 |

In general, the compounds according to the invention are non-toxic at therapeutically useful doses. Thus for example the compound of Examples 6, 7, 8 and 10 produced no adverse effects when administered orally to guinea pigs at a dose of 5 mg/kg.

**Claims for the Contracting States: BE CH DE FR GB IT LI LU NL SE**

1. Compounds of the general formula (I)

$$QNH\overset{R^1}{\underset{R^2}{C}}(CH_2)_mO(CH_2)_nAr \qquad (I)$$

wherein

Ar represents a phenyl group optionally substituted by one or two substituents selected from halogen atoms, or $C_{1-3}$alkyl or $C_{1-3}$alkoxy groups, or by an alkylenedioxy group of formula $-O(CH_2)_pO-$ where p is 1 or 2;

$R^1$ and $R^2$ each represents a hydrogen atom or a $C_{1-3}$alkyl group with the proviso that the sum total of carbon atoms in $R^1$ and $R^2$ is not more than 4;

m is an integer from 2 to 8 and

n is an integer from 1 to 7 with the proviso that the sum total of m + n is 4 to 12;

Q represents a group of formula

$$R^a\underset{OH}{C}H\overset{R^3}{C}H- \text{ or } R^b\underset{OH}{C}HCH_2-,$$

where $R^3$ represents a hydrogen atom or a $C_{1-3}$alkyl group, $R^a$ represents:

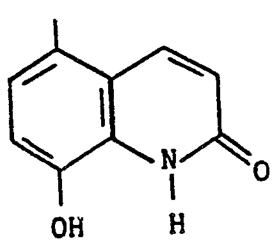

or

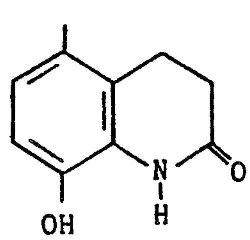

,

and $R^b$ represents:

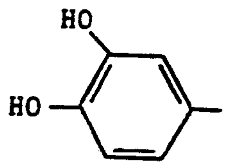 , 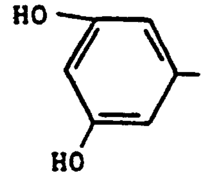 or 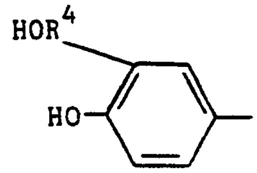

where $R^4$ represents a straight or branched $C_{2-3}$alkylene chain; and physiologically acceptable salts and solvates thereof.

2. Compounds as claimed in claim 1 in which the total number of carbon atoms in the chains $-(CH_2)_m-$ and $-(CH_2)_n-$ is 6 to 12 inclusive.

3. Compounds as claimed in claim 1 or 2, in which the chain $-(CH_2)_m-$ is $-(CH_2)_3-$, $-CH_2)_4-$, $-(CH_2)_5-$, $-(CH_2)_6-$ or $-(CH_2)_7-$, and the chain $-(CH_2)_n-$ is $-(CH_2)_2-$, $-(CH_2)_3-$, $-(CH_2)_4-$, $-(CH_2)_5-$ or $-(CH_2)_6-$.

4. Compounds as claimed in any of claims 1 to 3, in which $R^1$ and $R^2$ are each methyl, ethyl, propyl or isopropyl groups except that if one of $R^1$ and $R^2$ is a propyl or isopropyl group, then the other is a hydrogen atom or a methyl group.

5. Compounds as claimed in any of claims 1 to 4, in which Ar represents a phenyl group substituted by chlorine, bromine, iodine, fluorine, methyl, ethyl, methoxy or ethoxy or Ar represents an unsubstituted phenyl group.

6. Compounds as claimed in any of claims 1 to 5, in which in the definition of Q, the group $R^3$ is a hydrogen atom or a methyl, ethyl, propyl or isopropyl group and the chain $R^4$, if present, is $-(CH_2)_2-$, $-(CH_2)_3-$ or

$$-\underset{\displaystyle CH_3}{\underset{|}{CH}}-$$

7. Compounds as claimed in claim 1 which are:—
2-Hydroxy-5-[1-hydroxy-2-[[6-[2-(4-methoxyphenyl)ethoxy]hexyl]amino]ethyl]benzeneethanol;
2-Hydroxy-5-[1-hydroxy-2-[[1-methyl-6-(2-phenylethoxy)hexyl]amino]ethyl]benzeneethanol;
5-[1-Hydroxy-2-[[1-methyl-6-(2-phenylethoxy)hexyl]amino]ethyl]-1,3-benzenediol;
5-[1-Hydroxy-2-[[6-(4-phenylbutoxy)hexyl]amino]ethyl]-1,3-benzenediol;
and the physiologically acceptable salts and solvates thereof.

8. A process for the preparation of compounds as claimed in any of claims 1 to 7 or a physiologically acceptable salt or solvate thereof which comprises:

(1) reacting an amine of general formula (II)

$$YNH\overset{\displaystyle R^1}{\overset{|}{\underset{\displaystyle R^2}{\underset{|}{C}}}}(CH_2)_mO(CH_2)_nAr \qquad (II)$$

(wherein Y is a hydrogen atom or a group convertible thereto by catalytic hydrogenation) with a compound of formula

$$R^aCH\!-\!CHR^3 \text{ (epoxide, O)},\quad R^aCHCHR^3L \text{ (OH)},\quad R^bCH\!-\!CH_2 \text{ (epoxide, O)} \text{ or } R^bCHCH_2L \text{ (OH)}$$

(wherein L represents a leaving group) followed, if necessary, by removal of any protecting groups present; or

(2a) for the preparation of a compound of formula (I) in which $R^1$ is a hydrogen atom, alkylating an amine of general formula (III)

$$QNR^5R^6 \qquad (III)$$

(wherein $R^5$ is a hydrogen atom or a protecting group and $R^6$ is a hydrogen atom) with an alkylating agent of general formula (IV)

$$L\underset{\displaystyle R^2}{\underset{|}{C}}H(CH_2)_mO(CH_2)_nAr \qquad (IV)$$

(wherein L is a leaving group) followed, if necessary, by removal of any protecting group present; or

(2b) for the preparation of a compound of formula (I) in which $R^1$ represents a hydrogen atom, alkylating an amine of general formula (III) in which $R^5$ is a hydrogen atom or a protecting group and $R^6$ is a hydrogen atom or a group convertible thereto under the reaction conditions, with a compound of general formula (V)

$$R^2CO(CH_2)_mO(CH_2)_nAr \qquad (V)$$

in the presence of a reducing agent followed, if necessary, by removal of any protecting groups present; or

(3a) reducing a ketone of formula (VIa) or (VIb)

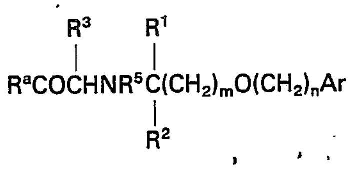

(VIa)

,

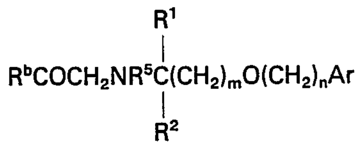

(VIb)

(wherein $R^5$ is a hydrogen atom or a protecting group) followed, if necessary, by removal of any protecting group present; or

(3b) for the preparation of a compound of general formula (I) in which $R^4$ is $-CH(CH_3)-$ reacting an aldehyde of general formula (VII)

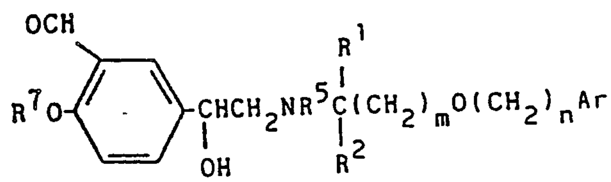

(VII)

(wherein $R^5$ and $R^7$ are each a hydrogen atom or a protecting group) with a Grignard reagent followed, if necessary, by removal of any protecting group present; or

(4) for the preparation of a compound of formula (I) in which $R^a$ is the group

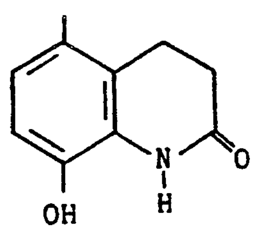

,

reducing the corresponding compound in which $R^a$ is the group

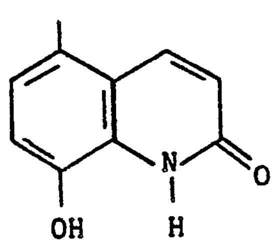

;

and if desired, converting the resulting compound of general formula (I) or a salt thereof into a physiologically acceptable salt or solvate thereof.

9. A pharmaceutical composition comprising at least one compound of general formula (I) as defined

in claim 1 or a physiologically acceptable salt or solvate thereof, together with a physiologically acceptable carrier or excipient.

**Claims for the Contracting State: AT**

1. A process for the production of compounds of the general formula (I)

$$\begin{array}{c}R^1\\ |\\ QNHC(CH_2)_mO(CH_2)_nAr\\ |\\ R^2\end{array} \qquad (I)$$

wherein

Ar represents a phenyl group optionally substituted by one or two substituents selected from halogen atoms, or $C_{1-3}$alkyl or $C_{1-3}$alkoxy groups, or by an alkylenedioxy group of formula $—O(CH_2)_pO—$ where p is 1 or 2;

$R^1$ and $R^2$ each represents a hydrogen atom or a $C_{1-3}$alkyl group with the proviso that the sum total of carbon atoms in $R^1$ and $R^2$ is not more than 4;

m is an integer from 2 to 8 and

n is an integer from 1 to 7 with the proviso that the sum total of m + n is 4 to 12;

Q represents a group of formula

$$\begin{array}{c}R^3\\ |\\ R^aCHCH—\\ |\\ OH\end{array} \text{ or } \begin{array}{c}R^bCHCH_2—,\\ |\\ OH\end{array}$$

where $R^3$ represents a hydrogen atom or a $C_{1-3}$alkyl group, $R^a$ represents:

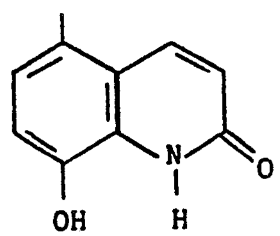

or

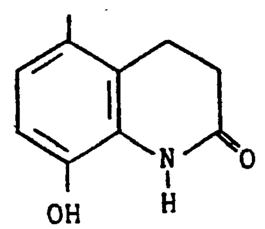

,

and $R^b$ represents:

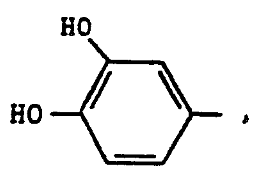

,

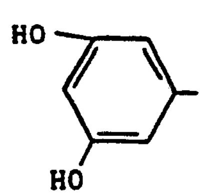

or

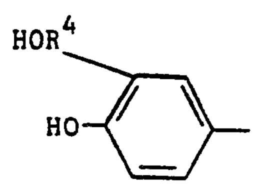

where $R^4$ represents a straight or branched $C_{2-3}$alkylene chain;

and physiologically acceptable salts and solvates thereof which comprises:

(1) reacting an amine of general formula (II)

$$\begin{array}{c}R^1\\ |\\ YNHC(CH_2)_mO(CH_2)_nAr\\ |\\ R^2\end{array} \qquad (II)$$

(wherein Y is a hydrogen atom or a group convertible thereto by catalytic hydrogenation) with a compound of formula

$$\underset{\diagdown O \diagup}{R^aCH—CHR^3}, \quad \underset{OH}{R^aCHCHR^3L}, \quad \underset{\diagdown O \diagup}{R^bCH—CH_2} \text{ or } \underset{OH}{R^bCHCH_2L}$$

(wherein L represents a leaving group) followed, if necessary, by removal of any protecting groups present; or

(2a) for the preparation of a compound of formula (I) in which $R^1$ is a hydrogen atom, alkylating an amine of general formula (III)

$$QNR^5R^6 \quad (III)$$

(wherein $R^5$ is a hydrogen atom or a protecting group and $R^6$ is a hydrogen atom) with an alkylating agent of general formula (IV)

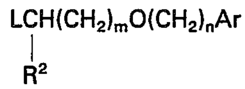

(IV)

(wherein L is a leaving group) followed, if necessary, by removal of any protecting group present; or

(2b) for the preparation of a compound of formula (I) in which $R^1$ represents a hydrogen atom, alkylating an amine of general formula (III) in which $R^5$ is a hydrogen atom or a protecting group and $R^6$ is a hydrogen atom or a group convertible thereto under the reaction conditions, with a compound of general formula (V)

$$R^2CO(CH_2)_mO(CH_2)_nAr \quad (V)$$

in the presence of a reducing agent followed, if necessary, by removal of any protecting groups present; or

(3a) reducing a ketone of formula (VIa) or (VIb)

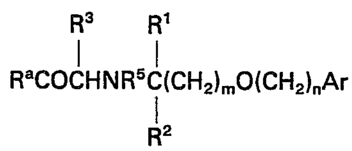

(VIa)

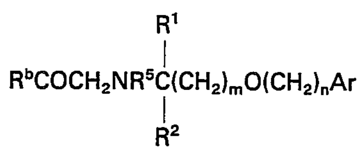

(VIb)

(wherein $R^5$ is a hydrogen atom or a protecting group) followed, if necessary, by removal of any protecting group present; or

(3b) for the preparation of a compound of general formula (I) in which $R^4$ is —$CH(CH_3)$— reacting an aldehyde of general formula (VII)

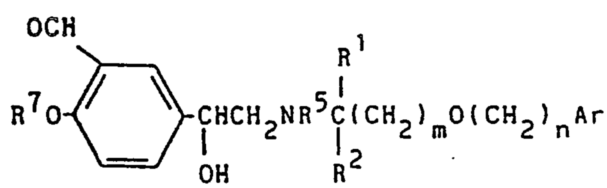

(VII)

(wherein $R^5$ and $R^7$ are each a hydrogen atom or a protecting group) with a Grignard reagent followed, if necessary, by removal of any protecting group present; or

(4) for the preparation of a compound of formula (I) in which $R^a$ is the group

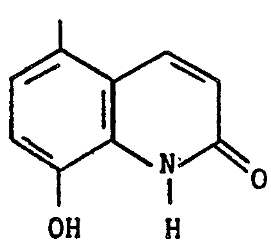

reducing the corresponding compound in which $R^a$ is the group

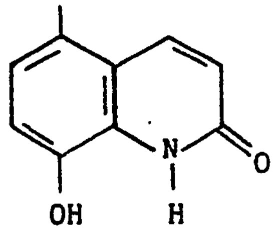 ;

and if desired, converting the resulting compound of general formula (I) or a salt thereof into a physiologically acceptable salt or solvate thereof.

2. A process as claimed in claim 1 for the production of compounds in which the total number of carbon atoms in the chains $—(CH_2)_m—$ and $—(CH_2)_n—$ is 6 to 12 inclusive.

3. A process as claimed in claim 1 or 2, for the production of compounds in which the chain $—(CH_2)_m—$ is $—(CH_2)_3—$, $—CH_2)_4—$, $—(CH_2)_5—$, $—(CH_2)_6—$ or $—(CH_2)_7—$, and the chain $—(CH_2)_n—$ is $—(CH_2)_2—$, $—(CH_2)_3—$, $—(CH_2)_4—$, $—(CH_2)_5—$ or $—(CH_2)_6—$.

4. A process as claimed in any of claims 1 to 3, for the production of compounds in which $R^1$ and $R^2$ are each methyl, ethyl, propyl or isopropyl groups except that if one of $R^1$ and $R^2$ is a propyl or isopropyl group, then the other is a hydrogen atom or a methyl group.

5. A process as claimed in any of claims 1 to 4, for the production of compounds in which Ar represents a phenyl group substituted by chlorine, bromine, iodine, fluorine, methyl, ethyl, methoxy or ethoxy or Ar represents an unsubstituted phenyl group.

6. A process as claimed in any of claims 1 to 5, for the production of compounds in which the definition of Q, the group $R^3$ is a hydrogen atom or a methyl, ethyl, propyl or isopropyl group and the chain $R^4$, if present, is $—(CH_2)_2—$, $—(CH_2)_3—$ or

$$—\underset{\displaystyle CH_3}{\underset{|}{CH}}—$$

7. A process as claimed in claim 1 for the production of compounds which are:—
2-Hydroxy-5-[1-hydroxy-2-[[6-[2-(4-methoxyphenyl)ethoxy]hexyl]amino]ethyl]benzeneethanol;
2-Hydroxy-5-[1-hydroxy-2-[[1-methyl-6-(2-phenylethoxy)hexyl]amino]ethyl]benzeneethanol;
5-[1-Hydroxy-2-[[1-methyl-6-(2-phenylethoxy)hexyl]amino]ethyl]-1,3-benzenediol;
5-[1-Hydroxy-2-[[6-(4-phenylbutoxy)hexyl]amino]ethyl]]-1,3-benzenediol;
and the physiologically acceptable salts and solvates thereof.

**Patentansprüche für die Vertragsstaaten: BE CH DE FR GB IT LI LU NL SE**

1. Verbindung der allgemeinen Formel (I)

$$QNH\overset{\displaystyle R^1}{\underset{\displaystyle R^2}{C}}(CH_2)_mO(CH_2)_nAr \qquad (I)$$

worin

Ar ein Phenylgruppe, die gegebenenfalls durch einen oder zwei Substituenten, ausgewählt unter Halogenatomen oder $C_{1-3}$-Alkyl- oder $C_{1-3}$-Alkoxygruppen, oder durch eine Alkylendioxygruppe der Formel $—O(CH_2)_pO—$, worin p für 1 oder 2 steht, substituiert sein kann, bedeutet;

$R^1$ und $R^2$ je ein Wasserstoffatom oder eine $C_{1-3}$-Alkylgruppe bedeuten, mit der Maßgabe, daß die Gesamtsumme der Kohlenstoffatome in $R^1$ und $R^2$ nicht über 4 liegt;

m eine ganze Zahl von 2 bis 8 bedeutet; und

n eine ganze Zahl von 1 bis 7 bedeutet, mit der Maßgabe, daß die Gesamtsumme von m + n 4 bis 12 beträgt;

Q eine Gruppe der Formel

$$R^aCH\overset{\displaystyle R^3}{\underset{|}{\overset{|}{C}H}}— \text{ oder } R^bCHCH_2—,$$

$$\quad\;\; OH \qquad\qquad\qquad OH$$

bedeutet, worin $R^3$ ein Wasserstoffatom oder eine $C_{1-3}$-Alkylgruppe bedeutet, $R^a$:

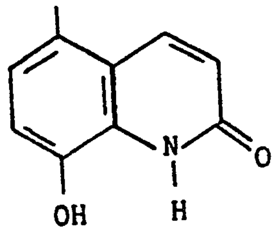

oder

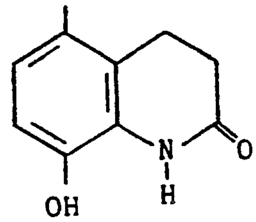

,

bedeutet und $R^b$:

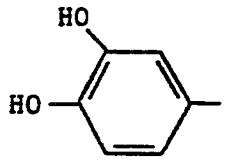

,

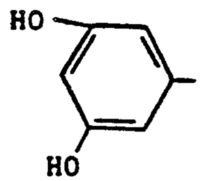

oder

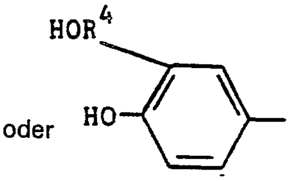

worin $R^4$ eine geradkettige oder verzweigte $C_{2-3}$-Alkylenkette bedeutet, bedeutet; und ihre physiologisch annehmbaren Salze und Solvate.

2. Verbindungen nach Anspruch 1, dadurch gekennzeichnet, daß die Gesamtanzahl der Kohlenstoffatome in den Ketten $-(CH_2)_m-$ und $-(CH_2)_n-$ 6 bis einschließlich 12 beträgt.

3. Verbindungen nach Anspruch 1 oder 2, dadurch gekennzeichnet, daß die $-(CH_2)_m$-Kette $-(CH_2)_3-$, $-CH_2)_4-$, $-(CH_2)_5-$, $-(CH_2)_6-$ oder $-(CH_2)_7-$, bedeutet und die $-(CH_2)_n-$ Kette $-(CH_2)_2-$, $-(CH_2)_3-$, $-(CH_2)_4-$, $-(CH_2)_5-$ oder $-(CH_2)_6-$ bedeutet.

4. Verbindungen nach einem der Ansprüche 1 bis 3, dadurch gekennzeichnet, daß $R^1$ und $R^2$ je Methyl-, Ethyl-, Propyl- oder Isopropylgruppen bedeuten, ausgenommen, daß, wenn einer der Substituenten $R^1$ und $R^2$ eine Propyl- oder Isopropylgruppe bedeutet, der andere ein Wasserstoffatom oder eine Methylgruppe bedeutet.

5. Verbindungen nach einem der Ansprüche 1 bis 4, dadurch gekennzeichnet, daß Ar eine durch Chlor, Brom, Iod, Fluor, Methyl, Ethyl, Methoxy oder Ethoxy substituierte Phenylgruppe bedeutet oder daß Ar eine unsubstituierte Phenylgruppe beduetet.

6. Verbindungen nach einem der Ansprüche 1 bis 5, dadurch gekennzeichnet, daß in der Definition von Q die Gruppe $R^3$ ein Wasserstoffatom oder eine Methyl-, Ethyl-, Propyl- oder Isopropylgruppe bedeutet und die Kette $R^4$, sofern vorhanden, $-(CH_2)_2-$, $-(CH_2)_3-$ oder

$$-\underset{\displaystyle CH_3}{\underset{|}{CH}}-$$

bedeutet.

7. Verbindungen nach Anspruch 1, nämlich:

2-Hydroxy-5-[1-hydroxy-2-[[6-[2-(4-methoxyphenyl)ethoxy]hexyl]amino]ethyl]benzolethanol;
2-Hydroxy-5-[1-hydroxy-2-[[1-methyl-6-(2-phenylethoxy)hexyl]amino]ethyl]benzolethanol;
5-[1-Hydroxy-2-[[1-methyl-6-(2-phenylethoxy)hexyl]amino]ethyl]-1,3-benzoldiol;
5-[1-Hydroxy-2-[[6-(4-phenylbutoxy)hexyl]amino]ethyl]-1,3-benzoldiol;
und ihre physioligisch annehmbaren Salze und Solvate.

8. Verfahren zur Herstellung von Verbindungen nach einem der Ansprüche 1 bis 7 oder eines ihrer physiologisch annehmbaren Salze oder Solvate, dadurch gekennzeichnet, daß

(1) eine Amin der allgemeinen Formel (II)

$$YNH\overset{\displaystyle R^1}{\overset{|}{\underset{\displaystyle R^2}{\underset{|}{C}}}}(CH_2)_mO(CH_2)_nAr \qquad (II)$$

(worin Y ein Wasserstoffatom oder eine durch katalytische Hydierung dazu umwandelbare Gruppe bedeutet) mit einer Verbindung der Formel

$$R^aCH\overset{O}{—}CHR^3,\quad R^aCH\underset{OH}{CH}R^3L,\quad R^bCH\overset{O}{—}CH_2 \text{ oder } R^bCH\underset{OH}{CH}_2L$$

(worin L eine abspaltbare Gruppe bedeutet) umgesetzt wird und gegebenenfalls irgendeine vorhandene Schultzgruppe entfernt wird; oder

(2a) für die Herstellung einer Verbindung der Formel (I), worin $R^1$ ein Wasserstoffatom bedeutet, ein Amin der allgemeinen Formel (III)

$$QNR^5R^6 \qquad (III)$$

(worin $R^5$ ein Wasserstoffatom oder eine Schutzgruppe bedeutet und $R^6$ ein Wasserstoffatom bedeutet) mit einem Alkylierungsmittel der allgemeinen Formel (IV)

$$\underset{R^2}{LCH}(CH_2)_mO(CH_2)_nAr \qquad (IV)$$

(worin L eine abspaltbare Gruppe bedeutet) umsetzt und gegebenenfalls irgendeine vorhandene Schutzgruppe entfernt; oder

(2b) für die Herstellung einer Verbindung der Formel (I), worin $R^1$ ein Wasserstoffatom bedeutet, eine Amin der allgemeinen Formel (III), worin $R^5$ ein Wasserstoffatom oder eine Schutzgruppe bedeutet und $R^6$ ein Wasserstoffatom oder eine darin bei den Reaktionsbedingungen umwåndelbare Gruppe bedeutet, mit Verbindungen der allgemeinen Formel (V)

$$R^2CO(CH_2)_mO(CH_2)_nAr \qquad (V)$$

in Anwesenheit eines Reduktionsmittels umsetzt und gegebenenfalls anschließend irgendeine vorhandene Schutzgruppe entfernt; oder

(3a) ein Keton der Formel (VIa) oder (VIb)

$$R^aCO\overset{R^3}{C}HNR^5\underset{R^2}{\overset{R^1}{C}}(CH_2)_mO(CH_2)_nAr \qquad (VIa)$$

$$R^bCOCH_2NR^5\underset{R^2}{\overset{R^1}{C}}(CH_2)_mO(CH_2)_nAr \qquad (VIb)$$

(worin $R^5$ ein Wasserstoffatom oder eine Schutzgruppe bedeutet) reduziert und gegebenenfalls anschließend irgendeine vorhandene Schutzgruppe entfernt; oder

(3b) für die Herstellung einer Verbindung der allgemeinen Formel (I), worin $R^4$ —$CH(CH_3)$— bedeutet, einen Aldehyd der allgemeinen Formel (VII)

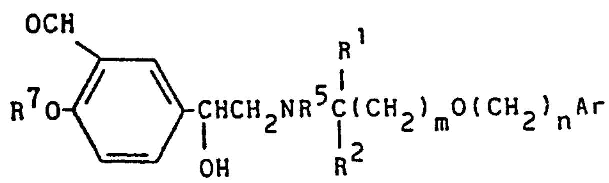

(VII)

(worin $R^5$ und $R^7$ je ein Wasserstoffatom oder eine Schultzgruppe bedeuten) mit einem Grignard-Reagens umsetzt und gegebenenfalls anschließend irgendeine vorhandene Schutzgruppe entferent; oder

(4) für die Herstellung einer Verbindung der Formel (I), worin $R^a$ die Gruppe

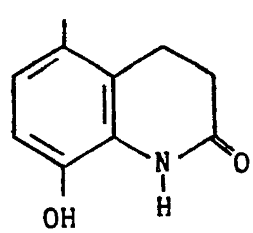

,

bedeutet, die entsprechende Verbindung, worin $R^a$ die Gruppe

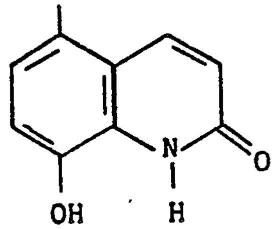

bedeutet, reduziert;
und gegebenenfalls die entstehende Verbindung der allgemeinen Formel (I) oder eines ihrer Salze in eines ihrer physiologisch annehmbaren Salze oder Solvate überführt.

9. Pharmazeutisches Präparat, dadurch gekennzeichnet, daß es mindestens eine Verbindung der allgemeinen Formel (I) nach Anspruch 1 oder eines seiner physioligisch annehmbaren Salze oder Solvate zusammen mit einem physioligisch annehmbaren Träger oder Arzneimittel- Verdünnungsstoff enthält.

**Patentansprüche für den Vertragsstaat: AT**

1. Verfahren zur Herstellung von Verbindungen der allgemeinen Formel (I)

$$QNHC(R^1)(R^2)(CH_2)_mO(CH_2)_nAr \qquad (I)$$

worin

Ar ein Phenylgruppe, die g egebenenfalls durch einen oder zwei Substituenten, ausgewählt unter Halogenatomen oder $C_{1-3}$-Alkyl- oder $C_{1-3}$-Alkoxygruppen, oder durch eine Alkylendioxygruppe der Formel $—O(CH_2)_pO—$, worin p für 1 oder 2 steht, substituiert sein kann, bedeutet;

$R^1$ und $R^2$ je ein Wasserstoffatom oder eine $C_{1-3}$-Alkylgruppe bedeuten, mit der Maßgabe, daß die Gesamtsumme der Kohlenstoffatome in $R^1$ und $R^2$ nicht über 4 liegt;

m eine ganze Zahl von 2 bis 8 bedeutet; und

n eine ganze Zahl von 1 bis 7 bedeutet, mit der Maßgabe, daß die Gesamtsumme von m + n 4 bis 12 beträgt;

Q eine Gruppe der Formel

$$R^aCH(OH)CH(R^3)— \text{ oder } R^bCH(OH)CH_2—,$$

bedeutet, worin $R^3$ ein Wasserstoffatom oder eine $C_{1-3}$-Alkylgruppe bedeutet, $R^a$:

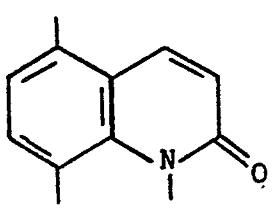

oder

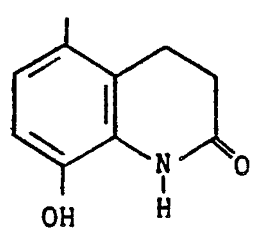

,

bedeutet und $R^b$:

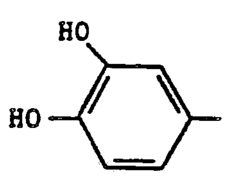

,

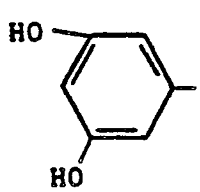

oder

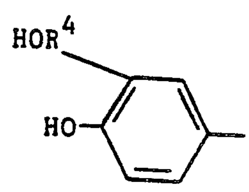

worin $R^4$ eine geradkettige oder verzweigte $C_{2-3}$-Alkylenkette bedeutet, bedeutet; und ihre physioligisch

annehmbaren Salze und Solvate, dadurch gekennzeichnet, daß

(1) eine Amin der allgemeinen Formel (II)

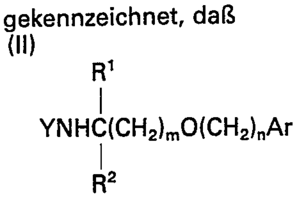 (II)

(worin Y ein Wasserstoffatom oder eine durch katalytische Hydierung dazu umwandelbare Gruppe bedeutet) mit einer Verbindung der Formel

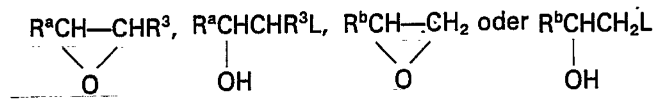

(worin L eine abspaltbare Gruppe bedeutet) umgesetzt wird und gegebenenfalls anschließend irgendeine vorhandene Schutzgruppe entfernt wird; oder

(2a) für die Herstellung einer Verbindung der Formel (I), worin $R^1$ ein Wasserstoffatom bedeutet, ein Amin der allgemeinen Formel (III)

$$QNR^5R^6 \qquad (III)$$

(worin $R^5$ ein Wasserstoffatom oder eine Schutzgruppe bedeutet und $R^6$ ein Wasserstoffatom bedeutet) mit einem Alkylierungsmittel der allgemeinen Formel (IV)

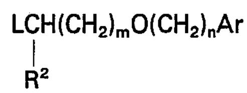 (IV)

(worin L eine abspaltbare Gruppe bedeutet) umsetzt und gegebenenfalls anschließend irgendeine vorhandene Schutzgruppe entfernt; oder

(2b) für die Herstellung einer Verbindung der Formel (I), worin $R^3$ ein Wasserstoffatom bedeutet, eine Amin der allgemeinen Formel (III), worin $R^5$ ein Wasserstoffatom oder eine Schutzgruppe bedeutet und $R^6$ ein Wasserstoffatom oder eine darin bei den Reaktionsbedingungen unwandelbare Gruppe bedeutet, mit Verbindungen der allgemeinen Formel (V)

$$R^2CO(CH_2)_mO(CH_2)_nAr \qquad (V)$$

in Anwesenheit eines Reduktionsmittels umsetzt und gegebenenfalls anschließend irgendeine vorhandene Schutzgruppe entfernt; oder

(3a) ein Keton der Formel (VIa) oder (VIb)

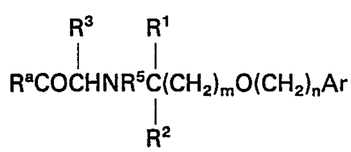 (VIa)

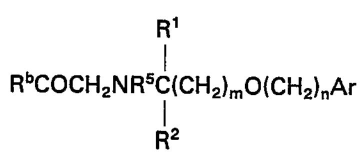 (VIb)

(worin $R^5$ ein Wasserstoffatom oder eine Schutzgruppe bedeutet) reduziert und gegebenenfalls anschließend irgendeine vorhandene Schutzgruppe entfernt; oder

(3b) für die Herstellung einer Verbindung der allgemeinen Formel (I), worin $R^4$ $—CH(CH_3)$- bedeutet, einen Aldehyd der allgemeinen Formel (VII)

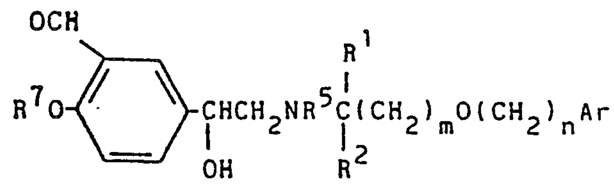

(VII)

(worin $R^5$ und $R^7$ je ein Wasserstoffatom oder eine Schultzgruppe bedeuten) mit einem Grignard-Reagens umsetzt und gegebenenfalls anschließend irgendeine vorhandene Schutzgruppe entferent; oder

(4) für die Herstellung einer Verbindung der Formel (I), worin $R^a$ die Gruppe

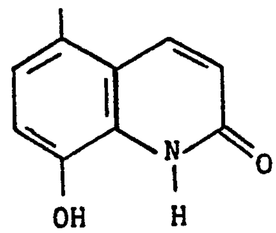

bedeutet, die entsprechende Verbindung, worin $R^a$ die Gruppe

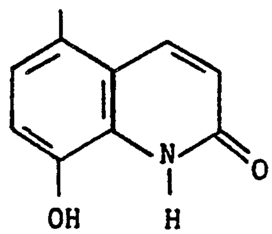

bedeutet, reduziert;

und gegebenenfalls die entstehende Verbindung der allgemeinen Formel (I) oder eines ihrer Salze in eines ihrer physiologisch annehmbaren Salze oder Solvate überführt.

2. Verfahren nach Anspruch 1, dadurch gekennzeichnet, daß Verbindungen hergestellt werden, worin die Gesamtzahl der Kohlenstoffatome in den Ketten $—(CH_2)_m—$ und $—(CH_2)_n—$ 6 bis einschließlich 12 beträgt.

3. Verfahren nach Anspruch 1 oder 2, dadurch gekennzeichnet, daß Verbindungen hergestellt werden, worin die $—(CH_2)_m$-Kette $—(CH_2)_3—$, $—(CH_2)_4—$, $—(CH_2)_5—$, $—(CH_2)_6—$ oder $—(CH_2)_7—$, bedeutet und die $—(CH_2)_n—$ Kette $—(CH_2)_2—$, $—(CH_2)_3—$, $—(CH_2)_4—$, $—(CH_2)_5—$ oder $—(CH_2)_6—$ bedeutet.

4. Verfahren nach einem der Ansprüche 1 bis 3, dadurch gekennzeichnet, daß Verbindungen hergestellt werden, worin $R^1$ und $R^2$ je Methyl-, Ethyl-, Propyl- oder Isopropylgruppen bedeuten, ausgenommen, daß, wenn einer der Substituenten $R^1$ und $R^2$ eine Propyl- oder Isopropylgruppe bedeutet, der andere ein Wasserstoffatom oder eine Methylgruppe bedeutet.

5. Verfahren nach einem der Ansprüche 1 bis 4, dadurch gekennzeichnet, daß Verbindungen hergestellt werden, worin Ar eine durch Chlor, Brom, Iod, Fluor, Methyl, Ethyl, Methoxy oder Ethoxy substituierte Phenylgruppe bedeutet oder worin Ar eine unsubstituierte Phenylgruppe beduetet.

6. Verfahren nach einem der Ansprüche 1 bis 5, dadurch gekennzeichnet, daß Verbindungen hergestellt werden worin der Definition von Q die Gruppe $R^3$ ein Wasserstoffatom oder eine Methyl-, Ethyl-, Propyl- oder Isopropylgruppe bedeutet und die Kette $R^4$, sofern vorhanden, $—(CH_2)_2—$, $—(CH_2)_3—$ oder

$$\begin{array}{c}—CH— \\ | \\ CH_3\end{array}$$

bedeutet.

7. Verfahren nach Anspruch 1, dadurch gekennzeichnet, daß die Verbindungen:
2-Hydroxy-5-[1-hydroxy-2-[[6-[2-(4-methoxyphenyl)ethoxy]hexyl]amino]ethyl]benzolethanol;
2-Hydroxy-5-[1-hydroxy-2-[[1-methyl-6-(2-phenylethoxy)hexyl]amino]ethyl]benzolethanol;
5-[1-Hydroxy-2-[[1-methyl-6-(2-phenylethoxy)hexyl]amino]ethyl]-1,3-benzoldiol;
5-[1-Hydroxy-2-[[6-(4-phenylbutoxy)hexyl]amino]ethyl]-1,3-benzoldiol;
und ihre physiologisch annehmbaren Salze und Solvate hergestellt werden.

**Revendications pour les Etats contractants: BE CH DE FR GB IT LI LU NL SE**

1. Composé de formule générale (I)

$$R^1$$
$$|$$
$$QNHC(CH_2)_mO(CH_2)_nAr \qquad (I)$$
$$|$$
$$R^2$$

dans laquelle

Ar représente un groupe phényle facultativement substitué par un ou deux substituants choisis parmi les atomes d'halogène ou les groupes alkyles en $C_{1-3}$ ou alcoxy en $C_{1-3}$, ou par un groupe alkylènedioxy de formule $—O(CH_2)_pO—$ dans laquelle p est 1 ou 2;

$R^1$ et $R^2$ représentant chacun un atome d'hydrogène ou un groupe alkyle en $C_{1-3}$, sous réserve que la somme totale des atomes de carbone de $R^1$ et $R^2$ ne dépasse pas 4;

m est un entier de 2 à 8 et

n est un entier de 1 à 7, sous réserve que la somme totale de m + n vaille 4 à 12;

Q représente un groupe de formule

$$R^3$$
$$|$$
$$R^aCHCH— \text{ ou } R^bCHCH_2—,$$
$$| \qquad\qquad |$$
$$OH \qquad\quad OH$$

où $R^3$ représente un atome d'hydrogène ou un group alkyle en $C_{1-3}$, $R^a$ représente:

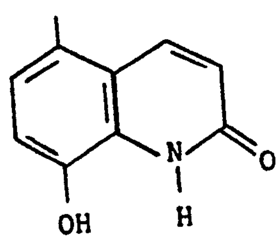

ou

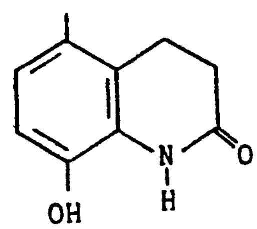

,

et $R^b$ représente:

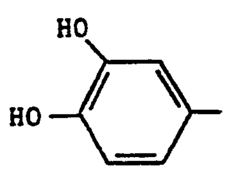

,

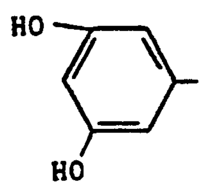

ou

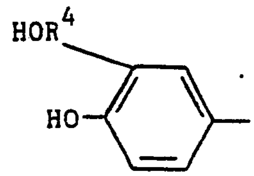

où $R^4$ représente une chaîne alkylène droite ou ramifiée en $C_{2-3}$;
et leurs sels convenant en pharmacie et leurs solvates.

2. Composés selon la revendication 1 où le nombre total des atomes de carbone dans les chaînes $—(CH_2)_m—$ et $—(CH_2)_n—$ est de 6 à 12 inclusivement.

3. Composés selon la revendication 1 ou 2, où la chaîne $—(CH_2)_m—$ est $—(CH_2)_3—$, $—CH_2)_4—$, $—(CH_2)_5—$, $—(CH_2)_6—$ ou $—(CH_2)_7—$, et la chaîne $—(CH_2)_n—$ est $—(CH_2)_2—$, $—(CH_2)_3—$, $—(CH_2)_4—$, $—(CH_2)_5—$ ou $—(CH_2)_6—$.

4. Composés selon l'une quelconque des revendications 1 à 3, où $R^1$ et $R^2$ sont chacun un groupe méthyle, éthyle, propyle ou isopropyle, si ce n'est que lorsqu'un de $R^1$ et $R^2$ est un groupe propyle ou isopropyle, l'autre est un atome d'hydrogène ou un groupe méthyle.

5. Composés selon l'une quelconque des revendications 1 à 4, où Ar représente un group phényle substitué par chlore, brome, iode, fluor, méthyle, éthyle, méthoxy ou éthoxy ou Ar représente un groupe phényle non substitué.

6. Composés selon l'une quelconque des revendications 1 à 5, où, dans la définition de Q, le groupe $R^3$ est un atome d'hydrogène ou un groupe méthyle, éthyle, propyle ou isopropyle et la chaîne $R^4$, lorsqu'elle est présente, est $—(CH_2)_2—$, $—(CH_2)_3—$ ou

$$—CH—$$
$$|$$
$$CH_3$$

7. Composés selon la revendication 1 qui sont:
le 2-hydroxy-5-[1-hydroxy-2-[[6-[2-(4-méthoxyphényl)éthoxy]hexyl]amino]éthyl]benzéne-éthanol;
le 2-hydroxy-5-[1-hydroxy-2-[[1-méthyl-6-(2-phényléthoxy)hexyl]amino]éthyl]benzéne-éthanol;
le 5-[1-hydroxy-2-[[1-methyl-6-(2-phényléthoxy)hexyl]amino]éthyl]-1,3-benzènediol;
le 5-[1-hydroxy-2-[[6-(4-phénylbutoxy)hexyl]amino]éthyl]-1,3-benzènediol;
et leurs sels convenant en physiologie et leurs solvates.

8. Procédé pour la préparation de composés selon l'une quelconque des revendications 1 à 7 ou d'un de leurs sels convenant en physioligie ou d'un de leurs solvates, qui comprend:

(1) la réaction d'une amine de formule générale (II)

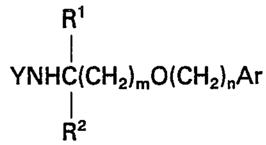

(II)

(dans laquelle Y est un atome d'hydrogène ou un groupe convertible en celui-ci par hydrogénation catalytique) avec un composé de formule

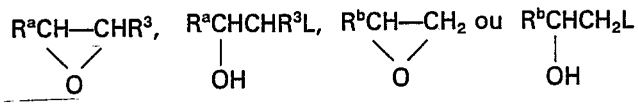

ou

(où L représente un groupe labile) suivie au besoin de l'élimination de tous les groupes protecteurs présents; ou

(2a) pour la préparation d'un composé de formule (I) dans laquelle $R^1$ est un atome d'hydrogène, l'alkylation d'une amine de formule générale (III)

$$QNR^5R^6 \qquad (III)$$

(dans laquelle $R^5$ est un atome d'hydrogène ou un groupe protecteur et $R^6$ est un atome d'hydrogène), avec un agent d'alkylation de formule générale (IV)

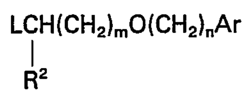

(IV)

(dans laquelle L est un groupe labile), suivie au besoin de l'élimination de tous les groupes protecteurs présents; ou

(2b) pour la préparation d'un composé de formule (I) dans laquelle $R^1$ représente un atome d'hydrogène, l'alkylation d'une amine de formule générale (III) dans laquelle $R^5$ est un atome d'hydrogéne ou un groupe protecteur et $R^6$ est un atome d'hydrogène ou un groupe convertible en celui-ci dans les conditions de réaction, avec un composé de formule générale (V)

$$R^2CO(CH_2)_mO(CH_2)_nAr \qquad (V)$$

en présence d'un agent réducteur, suivie au besoin de l'élimination de tous les groupes protecteurs présents; ou

(3a) la réduction d'une cétone de formule (VIa) ou (VIb)

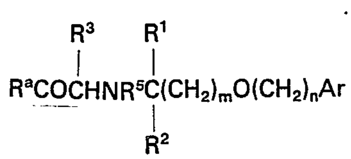

(VIa)

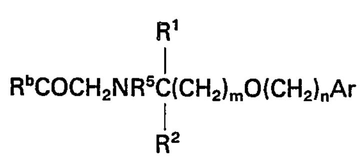

(VIb)

dans lesquelles $R^5$ est un atome d'hydrogène ou un groupe protecteur), suivie au besoin de l'élimination de tous les groupes protecterus présents; ou

(3b) pour la préparation d'un composé de formule générale (i) dans laquelle $R^4$ est $-CH(CH_3)-$, la réaction d'un aldéhyde de formule générale (VII)

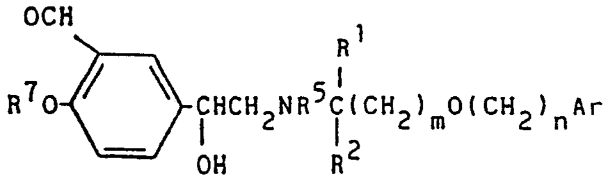

(VII)

(dans laquelle $R^5$ et $R^7$ sont chacun un atome d'hydrogène ou un groupe protecteur) avec un réactif de Grignard, suivie au besoin de l'élimination de tous les groupes protecteurs présents; ou

(4) pour la préparation d'un composé de formule (I) dans laquelle $R^a$ est le groupe

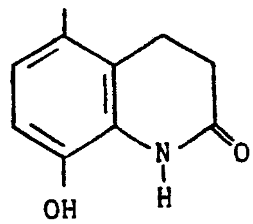

,

la réduction du composé correspondant dans lequel $R^a$ est le groupe

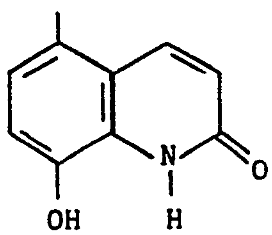

si on le désire, la conversion du composé obtenu de formule générale (I) ou d'un sel de celui-ci en un sel convenant en physiologie ou un solvate de clui-ci.

9. Composition pharmaceutique comprenant au moins un composé de formule générale (I) comme défini dans la revendication 1, ou un sel convenant en physiologie ou un solvate de celui-ci, avec un véhicule ou excipient convenant en physiologie.

**Revendications pour l'Etat contractant: AT**

1. Procédé pour la production de composés de formule générale (I)

$$QNH\overset{\overset{\displaystyle R^1}{|}}{\underset{\underset{\displaystyle R^2}{|}}{C}}(CH_2)_mO(CH_2)_nAr \qquad (I)$$

dans laquelle

Ar représente un groupe phényle facultativement substitué par un ou deux substituants choisis parmi les atomes d'halogène ou les groupes alkyles en $C_{1-3}$ ou alcoxy en $C_{1-3}$, ou par un groupe alkylènedioxy de formule $-O(CH_2)_pO-$ dans laquelle p est 1 ou 2;

$R^1$ et $R^2$ représentent chacun un atome d'hydrogène ou un groupe alkyle en $C_{1-3}$, sous réserve que la somme totale des atomes de carbone de $R^1$ et $R^2$ ne dépasse pas 4;

m est un entier de 2 à 8 et

n est un entier de 1 à 7, sous réserve que la somme totale de m + n vaille 4 à 12;

Q représente un groupe de formule

$R^3$
$R^aCHCH-$ ou $R^bCHCH_2-$,
OH OH

où $R^3$ représente un atome d'hydrogène ou un group alkyle en $C_{1-3}$, $R^a$ représente:

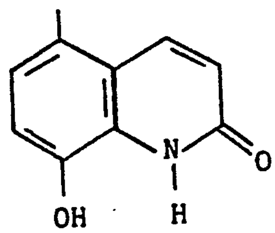
ou
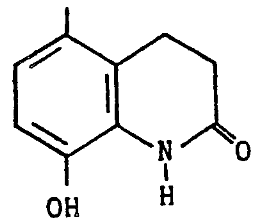
,

et $R^b$ représente:

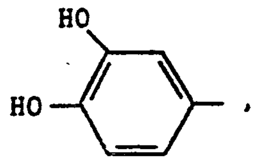
,
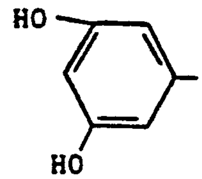
ou
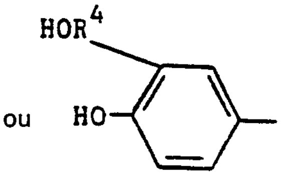

où $R^4$ représente une chaîne alkylène droite ou ramifiée en $C_{2-3}$;
et leurs sels convenant en pharmacie et leurs solvates, qui comprend:

(1) la réaction d'une amine de formule générale (II)

$R^1$
$YNHC(CH_2)_mO(CH_2)_nAr$ (II)
$R^2$

(dans laquelle Y est un atome d'hydrogène ou un groupe convertible en celui-ci par hydrogénation catalytique) avec un composé de formule

$R^aCH-CHR^3$, $R^aCHCHR^3L$, $R^bCH-CH_2$ ou $R^bCHCH_2L$
O OH O OH

(où L représente un groupe labile) suivie au besoin de l'élimination de tous les groupes protecteurs présents; ou

(2a) pour la préparation d'un composé de formule (I) dans laquelle $R^1$ est un atome d'hydrogène, l'alkylation d'une amine de formule générale (III)

$QNR^5R^6$ (III)

(dans laquelle $R^5$ est un atome d'hydrogène ou un groupe protecteur et $R^6$ est un atome d'hydrogène), avec un agent d'alkylation de formule générale (IV)

$LCH(CH_2)_mO(CH_2)_nAr$ (IV)
$R^2$

(dans laquelle L est un groupe labile), suivie au besoin de l'élimination de tous les groupes protecteurs présents; ou

(2b) pour la préparation d'un composé de formule (I) dans laquelle $R^1$ représente un atome d'hydrogène, l'alkylation d'une amine de formule générale (III) dans laquelle $R^5$ est un atome d'hydrogéne ou un groupe protecteur et $R^6$ est un atome d'hydrogène ou un groupe convertible en celui-ci dans les

conditions de réaction, avec un composé de formule générale (V)

$$R^2CO(CH_2)_mO(CH_2)_nAr \qquad (V)$$

en présence d'un agent réducteur, suivie au besoin de l'élimination de tous les groupes protecteurs présents; ou

(3a) la réduction d'une cétone de formule (VIa) ou (VIb)

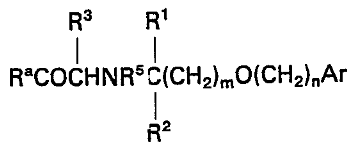

(VIa)

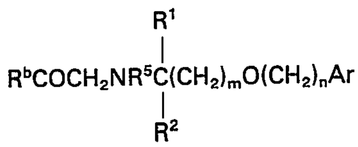

(VIb)

dans lesquelles $R^5$ est un atome d'hydrogène ou un groupe protecteur), suivie au besoin de l'élimination de tous les groupes protecteurs présents; ou

(3b) pour la préparation d'un composé de formule générale (I) dans laquelle $R^4$ est $—CH(CH_3)—$, la réaction d'un aldéhyde de formule générale (VII)

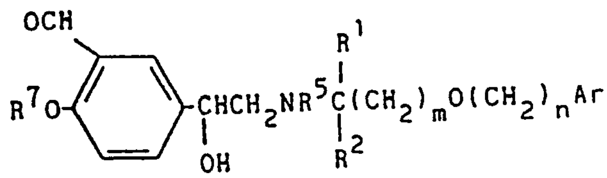

(VII)

(dans laquelle $R^5$ et $R^7$ sont chacun un atome d'hydrogène ou un groupe protecteur) avec un réactif de Grignard, suivie au besoin de l'élimination de tous les groupes protecteurs présents; ou

(4) pour la préparation d'un composé de formule (I) dans laquelle $R^a$ est le groupe

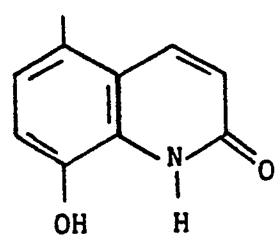

la réduction du composé correspondant dans lequel $R^a$ est le groupe

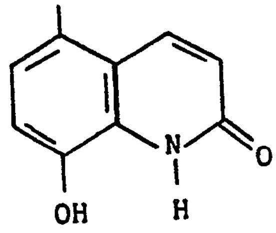

; et

si on le désire, la conversion du composé obtenu de formule générale (I) ou d'un sel de celui-ci en un sel convenant en physiologie ou un solvate de clui-ci.

2. Procédé selon la revendication 1 où la production de composés dans lesquels le nombre total des atomes de carbone dans les chaînes $—(CH_2)_m—$ et $—(CH_2)_n—$ est de 6 à 12 inclusivement.

3. Procédé selon la revendication 1 ou 2 pour la production de composés dans lesquels la chaîne

$-(CH_2)_m-$ est $-(CH_2)_3-$, $-(CH_2)_4-$, $-(CH_2)_5-$, $-(CH_2)_6-$ ou $-(CH_2)_7-$, et la chaîne $-(CH_2)_n-$ est $-(CH_2)_2-$, $-(CH_2)_3-$, $-(CH_2)_4-$, $-(CH_2)_5-$ ou $-(CH_2)_6-$.

4. Procédé selon l'une quelconque des revendications 1 à 3 pour la production de composés dans lesquels $R^1$ et $R^2$ sont chacun un groupe méthyle, éthyle, propyle ou isopropyle, si ce n'est que lorsqu'un de $R^1$ et $R^2$ est un groupe propyle ou isopropyle, l'autre est un atome d'hydrogène ou un groupe méthyle.

5. Procédé selon l'une quelconque des revendications 1 à 4 pour la production de composés dans lesquels Ar représente un group phényle substitué par chlore, brome, iode, fluor, méthyle, éthyle, méthoxy ou éthoxy ou Ar représente un groupe phényle non substitué.

6. Procédé selon l'une quelconque des revendications 1 à 5, pour la production de composés dans lesquels, dans la définition de Q, le groupe $R^3$ est un atome d'hydrogène ou un groupe méthyle, éthyle, propyle ou isopropyle et la chaîne $R^4$, lorsqu'elle est présente, est $-(CH_2)_2-$, $-(CH_2)_3-$ ou

$$-\underset{\displaystyle CH_3}{\underset{|}{CH}}-$$

7. Procédé selon la revendication 1 pour la production de composé qui sont:
le 2-hydroxy-5-[1-hydroxy-2-[[6-[2-(4-méthoxyphényl)éthoxy]hexyl]amino]éthyl]benzéne-éthanol;
le 2-hydroxy-5-[1-hydroxy-2-[[1-méthyl-6-(2-phényléthoxy)hexyl]amino]éthyl]benzéne-éthanol;
le 5-[1-hydroxy-2-[[1-methyl-6-(2-phényléthoxy)hexyl]amino]éthyl]-1,3-benzènediol;
le 5-[1-hydroxy-2-[[6-(4-phénylbutoxy)hexyl]amino]éthyl]-1,3-benzènediol;
et leurs sels convenant en physioligie et leurs solvates.